# EUROPEAN PATENT APPLICATION

(11) **EP 4 470 559 A1**
(43) Date of publication of application: **04.12.2024**
(21) Application number: 23177053.8
(22) Date of filing: 02.06.2023
(51) Int. Cl.: A61K 39/12, A61K 39/39, A61P 31/20, C07K 16/28

(54) **COMBINATION CONTAINING AN MRNA VACCINE AND AN IMMUNE MODULATING MRNA FOR IMPROVED IMMUNOGENICITY AND EFFICACY**

(71) Applicant: Imgen-T-Srl, 80010 Naples (IT)
(72) Inventor: SASSO, Emanuele, 81100 Caserta (CE) (IT); NICOSIA, Alfredo, 80010 Naples (NA) (IT)
(74) Representative: Engelhard, Markus

(57) **Abstract**

The present invention pertains to a combination comprising two or more mRNA molecules, or a single mRNA molecule encoding a first molecule, which is a therapeutic or immunogenic protein or peptide, and a second molecule, which is a protein or a peptide having the ability to modulate an immune response against the first molecule and/or the translation product of the first molecule, wherein the combination comprises at least one first mRNA molecule encoding a first molecule, which is a therapeutic or immunogenic protein or peptide, and at least one second mRNA molecule encoding a second molecule, which is a protein or a peptide having the ability to modulate an immune response against the first molecule and/or the translation product of the first molecule. The invention also relates to a host cell comprising a combination or the single mRNA molecule according to the present invention, a pharmaceutical composition comprising the combination, the single mRNA molecule, or the host cell of the present invention, and a vaccine comprising the combination, the single mRNA molecule, the host cell, or the pharmaceutical composition according to the present invention. Further provided is a kit comprising the combination, the single mRNA molecule, the host cell, the pharmaceutical composition, or the vaccine according to the present invention. The invention also pertains to the combination, the single mRNA molecule, the host cell, the pharmaceutical composition, the vaccine, or the kit according to the present invention, for use in medicine. Finally, the invention also relates to the combination, the single mRNA molecule, the host cell, the pharmaceutical composition, the vaccine, or the kit according to the present invention, for use in a method of diagnosis, prevention, and/or treatment of an infectious, a genetic, or a proliferative disease.

## Description

### FIELD OF THE INVENTION

The present invention pertains to a combination comprising two or more mRNA molecules, or a single mRNA molecule encoding a first molecule, which is a therapeutic or immunogenic protein or peptide, and a second molecule, which is a protein or a peptide having the ability to modulate an immune response against the first molecule and/or the translation product of the first molecule, wherein the combination comprises at least one first mRNA molecule encoding a first molecule, which is a therapeutic or immunogenic protein or peptide, and at least one second mRNA molecule encoding a second molecule, which is a protein or a peptide having the ability to modulate an immune response against the first molecule and/or the translation product of the first molecule. The invention also relates to a host cell comprising a combination or the single mRNA molecule according to the present invention, a pharmaceutical composition comprising the combination, the single mRNA molecule, or the host cell of the present invention, and a vaccine comprising the combination, the single mRNA molecule, the host cell, or the pharmaceutical composition according to the present invention. Further provided is a pharmaceutical combination, and a kit comprising the combination, the single mRNA molecule, the host cell, the pharmaceutical composition, the vaccine, or the pharmaceutical combination according to the present invention. The invention also pertains to the combination, the single mRNA molecule, the host cell, the pharmaceutical composition, the vaccine, the pharmaceutical combination, or the kit according to the present invention, for use in medicine. Finally, the invention also relates to the combination, the single mRNA molecule, the host cell, the pharmaceutical composition, the vaccine, the pharmaceutical combination, or the kit according to the present invention, for use in a method of diagnosis, prevention, and/or treatment of an infectious, a genetic, or a proliferative disease.

### DESCRIPTION

In vivo transfer of ribonucleic acids with biological activity or encoding therapeutic peptides are at the forefront of modem therapy. This includes gene therapy approaches and genetic vaccination. However, since the majority of biological processes are finely regulated in order to achieve optimal activity, in vivo transfer of the ribonucleic acids with biological activity only is unlikely to achieve optimal efficacy.

For example, adaptive immunity is elicited as a response to an antigen and this response is further modulated by other cellular factors that improve or reduce its activity. In the case of subunit vaccines (i.e.: recombinant proteins), optimal adaptive immunity is obtained by adding adjuvants such as aluminium salts, oil in water emulsion, and the more recently approved AS01, AS03, AS04, CpG ODN, and MF59. However, at variance with classical vaccines, to date, no adjuvant has been combined with clinically approved messenger RNA (mRNA) vaccines, possibly due to the detrimental effect of the adjuvant-induced innate response on the antigen expression driven by the mRNA. Therefore, adaptive immunity induced by mRNA vaccines may achieve suboptimal results in terms of potency, and/or quality, and/or longevity.

WO 2003/051401 (A2) describes a pharmaceutical composition comprising at least one mRNA, which contains at least one region that codes for an antigen from a tumor, combined with an aqueous solvent and preferably a cytokine, such as GM-CSF. The pharmaceutical composition is proposed to be used for therapy and/or prophylaxis against cancer.

WO 2006/008154 (A1) discloses an mRNA mixture for vaccinating against tumor diseases, wherein at least one type of mRNA contains at least one tumor antigen-coding region. At least one other mRNA contains at least one type of an immunogenic protein-coding region.

WO 2016/170176 (A1) relates to RNA containing compositions for use in the treatment or prophylaxis of tumor and/or cancer diseases, and to uses of the RNA containing compositions for the treatment or prophylaxis of tumor and/or cancer diseases.

The problem to be solved by the present invention is to improve the efficacy of genetic vaccine approaches. The inventors' solution to this problem is to contemporarily administer, together with the mRNA vaccine one (or more) other mRNA molecule(s) with direct or indirect immunomodulating activity (modulating RNA). This strategy is based on a combination containing a therapeutic RNA (the mRNA vaccine) and a modulating mRNA as a mixture of LNP formulations or co-formulated (both mRNA included in the same LNP particle), or a single mRNA molecule encoding a first molecule, which is a therapeutic or immunogenic protein or peptide, and a second molecule, which is a protein or a peptide having the ability to modulate an immune response against the first molecule and/or the translation product of the first molecule to allow a spatially and temporally coordinated in vivo expression of the two proteins (failure to do so resulting in reduced or abrogated modulation).

### BRIEF DESCRIPTION OF THE INVENTION

Generally, and by way of brief description, the main aspects of the present invention can be described as follows:

In a first aspect, the invention pertains to a combination comprising two or more mRNA molecules or a single mRNA molecule encoding a first molecule, which is a therapeutic or immunogenic protein or peptide, and a second molecule, which is a protein or a peptide having the ability to modulate an immune response against the first molecule and/or the translation product of the first molecule.

In a second aspect, the invention pertains to a host cell comprising the combination or the single mRNA molecule according to the first aspect of the present invention.

In a third aspect, the invention pertains to a pharmaceutical composition comprising the combination or the single mRNA molecule according to the first aspect of the present invention, or the host cell according to the second aspect of the present invention, and a pharmaceutical acceptable carrier, stabilizer and/or excipient.

In a fourth aspect, the invention pertains to a vaccine comprising the combination or the single mRNA molecule according to the first aspect of the present invention, the host cell according to the second aspect of the present invention, or the pharmaceutical composition according to the third aspect of the present invention.

In a fifth aspect, the invention pertains to a pharmaceutical combination.

In a sixth aspect, the invention pertains to a kit comprising the combination or the single mRNA molecule according to the first aspect of the present invention, the host cell according to the second aspect of the present invention, the pharmaceutical composition according to the third aspect of the present invention, the vaccine according to the fourth aspect of the present invention, or the pharmaceutical combination according to the fifth aspect of the present invention.

In a seventh aspect, the invention pertains to the combination or the single mRNA molecule according to the first aspect of the present invention, the host cell according to the second aspect of the present invention, the pharmaceutical composition according to the third aspect of the present invention, the vaccine according to the fourth aspect of the present invention, the pharmaceutical combination according to the fifth aspect of the present invention, or the kit according to the sixth aspect of the present invention, for use in medicine.

In an eighth aspect, the invention pertains to the combination or the single mRNA molecule according to the first aspect of the present invention, the host cell according to the second aspect of the present invention, the pharmaceutical composition according to the third aspect of the present invention, the vaccine according to the fourth aspect of the present invention, the pharmaceutical combination according to the fifth aspect of the present invention, or the kit according to the sixth aspect of the present invention, for use in a method of diagnosis, prevention, and/or treatment of an infectious, a genetic, or a proliferative disease.

### DETAILED DESCRIPTION OF THE INVENTION

In the following, the elements of the invention will be described. These elements are listed with specific embodiments, however, it should be understood that they may be combined in any manner and in any number to create additional embodiments. The variously described examples and preferred embodiments should not be construed to limit the present invention to only the explicitly described embodiments. This description should be understood to support and encompass embodiments which combine two or more of the explicitly described embodiments or which combine the one or more of the explicitly described embodiments with any number of the disclosed and/or preferred elements. Furthermore, any permutations and combinations of all described elements in this application should be considered disclosed by the description of the present application unless the context indicates otherwise.

In the first aspect, the invention pertains to a combination comprising two or more mRNA molecules, or a single mRNA molecule encoding a first molecule, which is a therapeutic or immunogenic protein or peptide, and a second molecule, which is a protein or a peptide having the ability to modulate an immune response against the first molecule and/or the translation product of the first molecule, wherein the combination comprises:
(i) at least one first mRNA molecule encoding a first molecule, which is a therapeutic or immunogenic protein or peptide, and
(ii) at least one second mRNA molecule encoding a second molecule, which is a protein or a peptide having the ability to modulate an immune response against the first molecule and/or the translation product of the first molecule,
wherein the at least one first mRNA molecule and the at least one second mRNA molecule are co-formulated into a single vector, or wherein the at least one first mRNA molecule and the at least one second mRNA molecule are formulated into two separate compositions, optionally wherein the two separate compositions are mixed prior to administration to form a combined composition, or wherein the first molecule and the second molecule are translated from the single mRNA molecule, for example by an internal ribosomal entry site (IRES) or by a self-cleaving peptide (such as a 2A peptide), optionally wherein the single mRNA molecule is a bicystronic or a polycystronic mRNA molecule, wherein the at least one second mRNA molecule or the part of the single mRNA molecule encoding the second molecule is selected from the group consisting of an mRNA molecule encoding a cell-bound (or membrane-anchored) agonistic antibody or agonistic antibody fragment, or an agonistic peptide against a cell-bound (or membrane-anchored) receptor or ligand, an mRNA molecule encoding for a cell-bound (or membrane-anchored) receptor or ligand, an mRNA molecule encoding for a chemokine or a cytokine, an mRNA molecule encoding for an antagonistic antibody or an antagonistic antibody fragment against an immune checkpoint protein, an mRNA molecule encoding for an immune checkpoint inhibitor, an mRNA molecule encoding for an immune checkpoint, and an mRNA molecule encoding for a transcription factor.

According to the present invention, the term "immune response" refers to either a specific reaction of the adaptive immune system to a particular antigen, which is then referred to as a specific or adaptive immune response, or an unspecific reaction of the innate immune system, which is then referred to as unspecific or innate immune response. Typically, the present invention is associated with adaptive immune responses. However, such adaptive immune responses can be supported by an additional innate immune response.

According to the present invention, the term "adaptive immune response" refers to an antigen-specific response of the immune system. Importantly, antigen specificity allows for the generation of responses that are tailored to specific pathogens or pathogen-infected cells, an ability that is usually maintained in the body by so-called "memory cells". In case a pathogen infects the body more than once, these specific memory cells are used to quickly eliminate the pathogen.

The term "adaptive immune system", as used herein, shall refer to a system that is essentially dedicated to eliminate or prevent pathogenic growth. It typically regulates the adaptive immune response by providing the vertebrate immune system with the ability to recognize and remember specific pathogens, thereby generating immunity, and to mount stronger attacks in each subsequent time a pathogen is encountered.

A "cellular immunity/cellular immune response", as used herein, usually comprises the activation of macrophages, natural killer cells (NK), antigen-specific cytotoxic T- lymphocytes, and the release of various cytokines in response to an antigen. In more general terms, cellular immunity is not based on antibodies, but on the activation of cells of the immune system. In one example, a cellular immune response may be characterized by activating antigen-specific cytotoxic T-lymphocytes that are able to induce apoptosis in cells, such as specific immune cells like dendritic cells or other cells, displaying epitopes of foreign antigens on their surface. Such cells may be virus-infected or infected with intracellular bacteria, or cancer cells displaying tumor antigens. Further characteristics of a cellular immune response may be the activation of macrophages and natural killer cells, enabling them to destroy pathogens and stimulation of cells to secrete a variety of cytokines that influence the function of other cells involved in adaptive immune responses and innate immune responses.

The term "bicystronic mRNA molecule" or "polycystronic mRNA molecule", as used herein, refers to an mRNA molecule that typically has two (bicistronic) or more (multicistronic) open reading frames (ORF). Contrary, a "monocistronic mRNA molecule" typically comprises only one open reading frame. An open reading frame in this context is a sequence of several nucleotide triplets (codons) that can be translated into a peptide or protein.

Importantly, the present invention allows a spatially and/or temporally coordinated *in vivo* expression of the at least one first molecule, which is a therapeutic or immunogenic protein or peptide, and the at least one second molecule, which is a protein or a peptide having the ability to modulate the immune response against the first molecule and/or the translation product of the first molecule. Thus, the at least one second molecule serves as a genetic adjuvant. The term "adjuvant", as used herein refers to a component that may modify, e.g. enhance, the effect of other agents, such as a vaccine. The term "adjuvant", as used herein is to be interpreted in a broad sense and refers to a broad spectrum of substances. Typically, these substances are able to increase the immunogenicity of antigens. For example, adjuvants may be recognized by the innate immune systems and, e.g., may elicit an innate immune response.

In a preferred embodiment, the at least one second mRNA molecule or the part of the single mRNA molecule encoding the second molecule is encoding a protein with a positive immunomodulating activity, such as a protein with the ability to increase the potency and/or the quality and/or the longevity of an immune response, or wherein the at least one second mRNA molecule or the part of the single mRNA molecule encoding the second molecule is encoding a protein with a negative immunomodulating activity, such as a protein with the ability to decrease the potency and/or the quality and/or the longevity of an immune response.

In one embodiment, the single vector comprising the at least one first mRNA molecule and the at least one second mRNA molecule, or the single mRNA molecule, is comprised in a nanoparticle, which is preferably a lipo-nanoparticle (LNP) or a non-lipid based nanoparticle (such as a nanoparticle composed of polymers), and/or wherein the two separate compositions comprising the at least one first mRNA molecule and the at least one second mRNA molecule, respectively, are selected from nanoparticles, synthetic particles for in vivo transduction, extracellular vesicles, and viral vectors, preferably wherein the nanoparticles are LNPs or non-lipid based nanoparticles (such as nanoparticles composed of polymers), wherein the LNPs are preferably composed of ionizable cationic lipid, neutral lipid (helper lipid), cholesterol, and PEGylated lipid, optionally wherein the molar lipid ratio (%) of ionizable cationic lipid:neutral lipid:cholesterol:PEG-ylated lipid in the composition is 50: 10:38.5: 1.5 or 46.3:9.4:42.7: 1.6, and wherein the N/P ratio is preferably, but not limited to, 3, 6, 4, or 8, wherein N is ionizable cationic lipid (nitrogen) and P is nucleotide (phosphate).

In a LNP according to the present invention, the at least one first mRNA molecule and the at least one second mRNA molecule, or the single mRNA molecule, is complexed with one or more lipids and thereby forming liposomes, lipid nanoparticles and/or lipoplexes.

In another embodiment, the at least one second mRNA molecule or the part of the single mRNA molecule encoding the second molecule is encoding:
i) a chemokine or a cytokine (either soluble or cell-bound or membrane-anchored) preferentially selected from but not restricted to the following list: Leptin, IL21, Ccl19, Ada, IL4, Ccl5, Cxcl9, Cxcl10, IL7, Ccl20, Ccl21a, Cxcl12, Ccl2, HMGB1, Ccl3, IL2, Efnb2, Ifng, IL12a, IL12b, IL1a, IL1b, IL36, TL1A, Cxcl13, Cxcl16, Ifnb1, IL13, IL17a, IL18, IL23a, IL6, Nlrp3, Ccl12, Ccl6, Ccl7, Ccl8, Chemerin, Csf2, Cxcl11, IL5, Ccl11, Ccl24, Ccl26, Ccl4, Csf1, IL11, Ccl17, Ccl22, Ccl25,Ccl9, Cxcl1, Cxcl14, Cxcl15, Cxcl2, Cxcl3, Cxcl5, IL22, IL3, Ccl27a, Ccl28, IL-12 single chain, IFNα, GM-CSF, IL15, IL-15sushi, and IL-15-receptor α chain chimeric protein, or
ii) a cell-bound (or membrane-anchored) ligand preferentially selected from but not restricted to the following list: LIGHT-Tnfsf14, OX40L-Tnfsf4, GITRL-Tnfsf18, 41BBL-Tnfsf9, Cd40lg, Cd70, ICOSL, Cd80, Icam1, Flt3l, CALR, Cd86, APRIL-Tnfsf13, CD166, LFA3, CD30L-Tnfsf8, Cd48, Cd74, Cd320, Cd83, Dpp4, Cd1d1, Cdld2, Clec4n, Clec5a, ITGAL, Slc11a1, Vcam1, CAV1, Cd14, PLXNB2, and TWEAK-Tnfsf12, or
iii) an agonistic antibody (preferably cell-bound or membrane-anchored) or an agonistic antibody fragment (preferably cell-bound or membrane-anchored) or an agonistic peptide (preferably cell-bound or membrane-anchored) having the same target of the molecules included in i) and/or ii).

In one particularly preferred embodiment relating to the first aspect of this invention, the at least one second mRNA molecule or the part of the single mRNA molecule encoding the second molecule is encoding a cell-bound (membrane-anchored) OX40 ligand, a cell-bound (membrane-anchored) ICOS ligand, a cell-bound (membrane-anchored) GITR ligand, a cell-bound (membrane-anchored) OX40-binding antibody, a cell-bound (membrane-anchored) ICOS-binding antibody, and/or a cell-bound (membrane-anchored) GITR-binding antibody.

In one embodiment relating to the first aspect of this invention, the at least one second mRNA molecule or the part of the single mRNA molecule encoding the second molecule is encoding a cell-bound (membrane-anchored) receptor or ligand with immunomodulatory activity from the TNF superfamily.

In another embodiment, the at least one second mRNA molecule or the part of the single mRNA molecule encoding the second molecule is encoding an antagonistic antibody or an antagonistic antibody fragment or an antagonistic peptide against an immune checkpoint protein, or an mRNA molecule encoding for an immune checkpoint inhibitor, or an mRNA molecule encoding for an immune checkpoint, and wherein the immune checkpoint protein is preferentially selected from but not restricted to any one of PD-1, cd47, cd31, PD-L1, PD-L2, BTLA, HVEM, lag3, tigit, prv, cd155, cd112, galectin-9, tim3, tim4, gitr, gitrl, tigit, Vista, cd276, cd39, cd73, CTLA4, IL10, IL35, ido, vip, IL27, and IL37.

Yet another embodiment relates to the combination or the single mRNA molecule according to the first aspect of this invention, wherein the at least one second mRNA molecule or the part of the single mRNA molecule encoding the second molecule is encoding a transcription factor, and wherein the transcription factor is preferentially selected from but not restricted to any one of Tcf1, TFEB, TFE3, NLRC5, CIITA, Lef1, Bcl11b, Gata3, Snai3, Ets1, IRF3, IRF7, Zbtb1, irf4, stat5b, runx3, bcl11a, foxo1, ikzf3, ets1, Tcf4, pax5, Bhlhe41, irf3, irf5, irf8, eomes, Stat1, Stat3, StatS, Cebpd, and Cebpa.

In a further embodiment, the at least one second mRNA molecule or the part of the single mRNA molecule encoding the second molecule is encoding for an antagonistic antibody or an antagonistic antibody fragment against an immune checkpoint protein or encoding an immune checkpoint inhibitor, and wherein:
(i) the antagonistic antibody or the antagonistic antibody fragment is targeting the PD-1/PD-L1 pathway, preferably wherein the mRNA molecule is encoding for an antibody selected from Atezolizumab, Avelumab, Cemiplimab, Dostarlimab, Durvalumab, Nivolumab, and Pembrolizumab;
(ii) the antagonistic antibody or the antagonistic antibody fragment is targeting the CTLA-4 pathway, preferably wherein the mRNA molecule is encoding for the antibody Ipilimumab, and/or
(iii) the antagonistic antibody or the antagonistic antibody fragment is targeting the LAG-3 pathway, preferably wherein the mRNA molecule is encoding for the antibody Relatlimab.

Atezolizumab (Tecentriq^{®}) is a checkpoint inhibitor that targets the PD-1/PD-L1 pathway; approved for subsets of patients with bladder cancer, breast cancer, liver cancer, lung cancer, and melanoma.

Avelumab (Bavencio^{®}) is a checkpoint inhibitor that targets the PD-1/PD-L1 pathway; approved for subsets of patients with bladder cancer, kidney cancer, and Merkel cell carcinoma, a type of skin cancer.

Cemiplimab (Libtayo^{®}) is a checkpoint inhibitor that targets the PD-1/PD-L1 pathway; approved for subsets of patients with cutaneous squamous cell carcinoma, basal cell carcinoma, and lung cancer.

Dostarlimab (Jemperli) is a checkpoint inhibitor that targets the PD-1 pathway; approved for subsets of patients with uterine (endometrial) cancer.

Durvalumab (Imfinzi^{™}) is a checkpoint inhibitor that targets the PD-1/PD-L1 pathway; approved for subsets of patients with bladder cancer and lung cancer.

Nivolumab (Opdivo^{®}) is a checkpoint inhibitor that targets the PD-1/PD-L1 pathway; approved for subsets of patients with bladder cancer, colorectal cancer, esophageal cancer, gastric cancer, head and neck cancer, kidney cancer, liver cancer, lung cancer, lymphoma, melanoma, and mesothelioma.

Pembrolizumab (Keytruda^{®}) is a checkpoint inhibitor that targets the PD-1/PD-L1 pathway; approved for subsets of patients with bladder cancer, breast cancer, cervical cancer, colorectal cancer, cutaneous squamous cell carcinoma, esophageal cancer, head and neck cancer, kidney cancer, liver cancer, lung cancer, lymphoma, melanoma, Merkel cell carcinoma, and stomach cancer. It is also approved to treat subsets of patients with cancers of any type that present with certain genetic mutations (MSI-H, dMMR, or TMB-H).

Ipilimumab (Yervoy^{®}) is a checkpoint inhibitor that targets the CTLA-4 pathway; approved for subsets of patients with melanoma, mesothelioma, liver cancer, and lung cancer.

Relatlimab is a checkpoint inhibitor that targets the LAG-3 pathway; approved in combination with nivolumab (together known as Opdualag^{™}) for subsets of patients with melanoma.

Yet another embodiment pertains to the combination or the single mRNA molecule according to the first aspect of this invention, wherein the at least one second mRNA molecule or the part of the single mRNA molecule encoding the second molecule is encoding a chemokine or a cytokine, and wherein:
(i) the cytokine targets the IL-2/IL-2R pathway, preferably wherein the mRNA molecule is encoding for the cytokine Aldesleukin;
(ii) the mRNA molecule is encoding for an immunomodulatory cytokine, such as Granulocytemacrophage colony-stimulating factor (GM-CSF), and/or
(iii) the cytokine targets the IFNAR1/2 pathway.

Aldesleukin (Proleukin^{®}) is a cytokine that targets the IL-2/IL-2R pathway; approved for subsets of patients with kidney cancer and melanoma.

In a further embodiment, the at least one first mRNA molecule or the part of the single mRNA molecule encoding the first molecule is encoding a therapeutic or immunogenic protein or peptide, which is preferably a therapeutic or immunogenic protein or peptide against an infectious disease antigen, such as an antigen selected from the group consisting of bacterial antigens, for example Gram-positive bacterial antigens or Gram-negative bacterial antigens, protozoan antigens, viral antigens, fungal antigens, and parasite antigens, or a cancer antigen, tumour antigen, and/or tumor neo-antigen, preferably selected from the group consisting of a single amino acid mutant peptide, a frame-shift peptide, a read-through mutation peptide, a splice site mutant peptide, and/or a senescence associated antigen, or wherein the at least one first mRNA molecule or the part of the single mRNA molecule encoding the first molecule is encoding a CAS protein, such as a CAS9 protein, or wherein the at least one first mRNA molecule or the part of the single mRNA molecule encoding the first molecule is encoding a therapeutic protein capable of correcting a disease due to a lack of a resident gene encoding the therapeutic protein or a mutation in the resident gene encoding the therapeutic protein or an inactivation of the resident gene encoding the therapeutic protein.

In the context of the present invention, the term "antigen", as used herein, shall refer to a peptide or a protein which may be recognized by the immune system, preferably by the adaptive immune system. An antigen is usually capable of triggering an antigen-specific immune response, e.g. by formation of antibodies and/or antigen-specific T cells as part of an adaptive immune response. An antigen usually comprises at least one epitope and which may be presented by MHC to T cells. In the sense of the present invention an antigen may be the product of translation of a provided RNA, preferably an mRNA as defined herein. In this context, also fragments, variants and derivatives of peptides and proteins comprising at least one epitope shall be understood as antigens. In the context of the present invention, therapeutic or immunogenic proteins or peptides against an infectious disease antigen, such as an antigen selected from the group consisting of bacterial antigens, protozoan antigens, viral antigens, fungal antigens, and parasite antigens, or a cancer antigen, tumour antigen, and/or tumor neo-antigen as defined herein is particularly preferred.

In one example, the at least one first mRNA molecule or the part of the single mRNA molecule encoding the first molecule is encoding at least one protein or peptide of one virus or bacterium of the following list: influenza virus type A or B or any other orthomyxovirus (influenza type C), picornaviruses, such as rhinovirus or hepatitis A virus, togaviruses, such as alphavirus or rubivirus, for example Sindbis, Semliki-Forest or rubeolavirus, rubella virus, coronaviruses, rhabdoviruses, such as rabies virus, paramyxoviruses, such as mumps virus, reoviruses, such as group A, B or C rotavirus, hepadnaviruses, such as hepatitis B virus, papoviruses, such as human papillomaviruses of any serotype, adenoviruses, herpesviruses, such as Herpes simplex virus 1, Herpes simplex virus 2 or Herpes simplex virus 3, cytomegalovirus, preferably CMVpp65, Epstein Barr virus, vacciniaviruses, the bacterium Chlamydophila pneumoniae, Flaviviruses, such as dengue virus type 1 to 4, yellow fever virus, West Nile virus, Japanese encephalitis virus, hepatitis C virus, caliciviruses, filoviruses, such as Ebola virus, bornaviruses, bunyaviruses, arenaviruses, such as lymphocytic choriomeningitis virus or hemorrhagic fever viruses, retroviruses, such as HIV, or parvoviruses.

Of note, another example of suboptimal activity achieved by mRNA vectors of the prior art may be exemplified by the excessive immune reactivity against the translation product of the *in vivo* delivered therapeutic mRNA, such as that elicited against peptides with molecular scissor activity (ie.: Cas9 or zincfinger nucleases), thereby restraining or reducing their activity and therapeutic efficacy. Similarly, *in vivo* delivery of mRNA encoding therapeutic proteins which is needed for substitution gene therapy may elicit an excessive adaptive immunity which limits the effectiveness of the therapy.

In one embodiment, the at least one first mRNA molecule or the part of the single mRNA molecule encoding the first molecule is encoding a Genome editing molecule/molecular scissor (such as a CAS protein like CAS9, or a Zn-finger nucleases).

Yet another embodiment relates to the combination or the single mRNA molecule according to the first aspect of this invention, wherein the at least one first mRNA molecule and the at least one second mRNA molecule, or the single mRNA molecule, is a synthetic and/or modified mRNA molecule, preferably wherein chemically modified nucleosides are incorporated, preferentially at a uracil nucleobase and/or at a cytidine nucleobase, in said at least one first mRNA molecule and said at least one second mRNA molecule, or in said single mRNA molecule, wherein every uracil nucleobase and/or every cytidine nucleobase in said at least one first mRNA molecule and said at least one second mRNA molecule, or in said single mRNA molecule, or a given percentage of uracil nucleobases and/or cytidine nucleobases in said at least one first mRNA molecule and said at least one second mRNA molecule, or in said single mRNA molecule, is replaced by chemically modified nucleosides, such as a N1-methyl-pseudouridine, pseudouridine (ψ), or 5-methylcytidine (m5C), and/or wherein the at least one first mRNA molecule and the at least one second mRNA molecule, or the single mRNA molecule, preferably include an optimized Kozak sequence, an optimized codon usage, optimized 5' and/or 3' untranslated sequences, a poly A tail, a 5' CAP preferentially selected from but not restricted to Anti-Reverse Cap Analog (ARCA), CAPO, CAP1: m7GpppNmpN, CAP2: m7GpppNmpNm, and/or an internal ribosomal entry site (IRES) in the context of linear or circular RNA.

As used herein, a "poly(A) tail" can also be referred to as "3 '-poly(A) tail", which is typically a long sequence of adenine nucleotides of up to about 400 adenosine nucleotides, such as from about 25 to about 400, preferably from about 50 to about 400, more preferably from about 50 to about 300, even more preferably from about 50 to about 250, most preferably from about 60 to about 250 adenosine nucleotides, added to the 3' end of a nucleic acid sequence, preferably an mRNA. A poly(A) tail may preferably be located 3' of the coding region comprised by a nucleic acid, e.g. an mRNA.

As used herein, a 5' Cap is typically a modified nucleotide, particularly a guanine nucleotide, added to the 5' end of an RNA molecule. In a preferred embodiment, the 5'-Cap is added using a 5 '-5 '-triphosphate linkage.

In another embodiment, the single vector comprising the at least one first mRNA molecule and the at least one second mRNA molecule, or each of the two separate compositions comprising the at least one first mRNA molecule and the at least one second mRNA molecule, or the single mRNA molecule, is a self-replicating RNA or a circular RNA.

In one embodiment, the single vector comprising the at least one first mRNA molecule and the at least one second mRNA molecule, or each of the two separate compositions comprising the at least one first mRNA molecule and the at least one second mRNA molecule, or the single mRNA molecule, is a nonviral RNA molecule.

In a further embodiment, the single vector comprising the at least one first mRNA molecule and the at least one second mRNA molecule, or each of the two separate compositions comprising the at least one first mRNA molecule and the at least one second mRNA molecule, or the single mRNA molecule, is a viral vector.

In the second aspect, the invention pertains a host cell comprising the combination or the single mRNA molecule according to the first aspect of this invention, optionally wherein the host cell is an antigen presenting cell, preferably a professional antigen presenting cell, or a lymphocyte, preferably a T lymphocyte or T lymphocyte progenitor, more preferably a CD4 or CD8 positive T-cell.

In the third aspect, the invention relates to a pharmaceutical composition comprising the combination or the single mRNA molecule according to the first aspect of this invention, or the host cell according to the second aspect of this invention, and a pharmaceutical acceptable carrier, stabilizer and/or excipient.

In the fourth aspect, the invention pertains to a vaccine comprising the combination or the single mRNA molecule according to the first aspect of this invention, or the host cell according to the second aspect of this invention, or the pharmaceutical composition according to the third aspect of this invention.

The term "vaccine", as used herein, typically refers to a prophylactic or therapeutic material providing at least one antigen, preferably an immunogen. "Providing at least on antigen" means, for example, that the vaccine comprises the antigen or that the vaccine comprises a molecule that, e.g., codes for the antigen or a molecule comprising the antigen. For example, the vaccine may comprise a nucleic acid, such as an RNA, which codes for a peptide or a protein that comprises the antigen. The antigen or immunogen may be derived from any material that is suitable for vaccination. For example, the antigen or immunogen may be derived from a pathogen, such as from bacteria or virus particles, or from a tumor or cancerous tissue. The antigen or immunogen stimulates the body's adaptive immune system to provide an adaptive immune response.

In a preferred embodiment, the vaccine is an RNA vaccine. The term "RNA vaccine", as used herein, is defined herein as a vaccine comprising at least one RNA molecule comprising at least one open reading frame (ORF) coding for at least one antigen. In the context of the present invention, the at least one RNA molecule comprised by the vaccine is preferably an isolated RNA molecule. This at least one RNA is preferably viral RNA, self-replicating RNA (replicon) or most preferably mRNA.

In one embodiment relating to the fourth aspect of this invention, the vaccine elicits an adaptive immune response, preferably a protective adaptive immune response against a virus, such as a virus selected from, but not restricted to, the following list: coronavirus, SARS coronavirus, SARS-CoV-2 coronavirus, respiratory syncytial virus (RSV), measles virus, influenza virus, rabies virus, Dengue fever virus, HIV (human immunodeficiency virus), hepatitis A virus (HAV), hepatitis B virus (HBV), hepatitis C virus (HCV), hepatitis D virus, hepatitis E virus, foot-and-mouth disease virus (FMDV), bovine leukosis virus (BLV), bovine parainfluenza virus, human parainfluenza virus (HPIV), bovine respiratory syncytial virus, porcine reproductive and respiratory syndrome virus, respiratory syncytial virus, vesicular stomatitis virus, bovine viral diarrhea virus, bovine coronavirus, BHV1 virus, equine arteritis virus, Nipah virus, BK virus, Porcine Respiratory Corona Virus Infection, Jaagsiekte Sheep Retrovirus, infectious bronchitis virus, avian pneumovirus, Calicivirus, enterovirus, Epstein-bar virus (EBV), newcastle disease virus, astrovirus, Influenzavirus A (Avian influenza) subtype H5N1, simple herpesvirus 1 (HSV-1), simple herpesvirus 2 (HSV-2), heartland virus, bocavirus (HBoV), human herpesvirus 6 (HHV-6), human herpesvirus 7 (HHV-7), human metapneumovirus (hMPV), human papilloma virus (HPV), Japanese encephalitis virus, JC virus, funin virus, Cytomegalovirus (CMV), Machupovirus, Marburg virus, infectious soft tumor virus (MCV), Lassavirus, epidemic parotitis virus, rhinovirus, rotavirus, varicella-zoster virus, Venezuelan encephalitis virus, West Nile virus, Western horse encephalitis virus, and Yellow fever virus, Filoviruses, and/or a bacterium, such as a bacterium selected from, but not restricted to, the following list: Acinetobacter, Acinetobacter baumannii, Actinobacillus, Anaplasma, Ancylostoma duodenale, hemolytic alkholderia Viralidae, Babesia, Bacillus, Bacillus anthracis, Bacillus cereus, Bacteroides, Burkholderi, Burkholderia henselae, Burkholderia mallei, Bordella pertussis, Bordetella, Brazilian burkholderia, Brucella, Campylobacter, Candida albicans, Chlamydia, Chlamydia trachomatis, Chlamydophila, Chlamydophila pneumoniae, Clostridium botulinum, Clostridium deficile, Clostridium candididium, Clostridium tetani, Corynebacterium diphtheriae, Clostridium, Clostridium burnetii, Cyanobacteria, Dientamoeba fragilis, Escherichia, Escherichia coli, Ehrlicia, Ehrlicia chafensis, Ehrlicia ewingii, Enterobacter, Enterococcus, Erwinia, Franciscella, Fusobacteria, Haemophilus, Haemophilus influenzae, Helicobacter, Hemophilus, Histoplasma capsultum, Kingera, Kingella, Kingella kingae, Klebsiella, Klebsiella granulomatis, Legionella, Legionella pneumophila, Listeria, Meningococcus, Moraxella, Mycobacterium leprae, Mycobacterium lepromatosis, Mycobacterium tuberculosis, Mycobacterium ulcerans mulcerans, Moraxella, pneumonia mycoplasma pneumoniae, Neisseria, Pasteurella, Prevotella, Proteus, Pseudomonas, Rickettsia, Salmonella, Serratia, Shigella, Staphylococcus, Streptococcus, Treponema, Vibrio, Yersinia, and ESKAPE pathogens, and/or a parasite, such as a parasite selected from, but not restricted to, the following list: Malaria, Leshmania, Cryptosoridium, Entamoeba, Taenia solium, Acaris lumbricoides, Echinococcus, Trypanosoma cruzi, Trypanosoma brucei, Schistosoma.

In another embodiment relating to the fourth aspect of this invention, the vaccine elicits an adaptive immune response, preferably a protective adaptive immune response against a cancer antigen or a tumour antigen, such as a cancer antigen or a tumour antigen selected from 1A01 HLA-A/m; 1A02; 5T4; ACRBP; AFP; AKAP4; alpha-actinin-4/m; alpha- methylacyl-coenzyme A _racemase; ANDR; ART-4; ARTCl/m; AURKB; B2MG; B3GN5; B4GN1; B7H4; BAGE-1; BASI; BCL-2; bcr/abl; beta-catenin/m; BING-4; BIRC7; BRCAl/m; BY55; calreticulin; CAMEL; CASPA; Caspase_8; cathepsin _B; cathepsin _L; CD1A; CD1B; CD1C; CD1D; CD1E; CD20; CD22; CD276; CD33; CD3E; CD3Z; CD4; CD44_Isoform _1; CD44_lsoform_6; CD52; CD55; CD56; CD80; CD86; CD8A; CDC27/m; CDE30; CDK4/m; CDKN2A/m; CEA; CEAM6; CH3L2; CLCA2; CML28; CML66; COA-1/m; coactosin-like_protein; collagen_XXIII; COX-2; CP1B1; CSAG2; CT-9/BRD6; CT45A1; CT55; CTAG2_Isoform_LAGE-1A; CTAG2_lsoform_LAGE-1B; CTCFL; Cten; cyclin_Bl; cyclin _D1; cyp-B; DAM-10; DEP1A; E7; EF1A2; EFTUD2/m; EGFR; EGLN3; ELF2/m; EMMPRIN; EpCam; EphA2; EphA3; ErbB3; ERBB4; ERG; ETV6; EWS; EZH2; FABP7; FCGR3A_Version_1; FCGR3A_Version_2; FGF5; FGFR2; fibronectin; FOS; FOXP3; FUT1; G250; GAGE-1; GAGE-2; GAGE-3; GAGE-4; GAGE-5; GAGE-6; GAGE7b; GAGE-8JGAGE-2D); GASR; GnT-V; GPC3; GPNMB/m; GRM3; HAGE; hepsin; Her2/neu; HLA-A2/m; homeobox_NKX3.1; HOM-TES-85; HPG1; HS71A; HS71B; HST-2; hTERT; iCE; IF2B3; IL-10; IL-13Ra2; IL2-RA; IL2-RB; IL2-RG; IL-5; IMP3; ITA5; ITB1; ITB6; kallikrein-2; kailikrein-4; KI20A; KIAA0205; KIF2C; KK-LC-1; LDLR; LGMN; LIRB2; LY6K; MAGA5; MAGA8; MAGAB; MAGE-_B1; MAGE-_E1; MAGE-A1; MAGE-A10; MAGE-A12; MAGE-A2; MAGE-A3; MAGE-A4; MAGE-A6; MAGE-A9; MAGE-BIO; MAGE-B16; MAGE-B17; MAGE-B2; MAGE-B3; MAGE-B4; MAGE-B5; MAGE-B6; MAGE-C1; MAGE-C2; MAGE-C3; MAGE-D1; MAGE-D2; MAGE-D4; MAGE-E1JMAGE1); MAGE-E2; MAGE-F1; MAGE-HI; MAGEL2; mammaglobin A; MART-l/melan-A; MART-2; MC1R; M-CSF; mesothelin; MITF; MMP1; MMP7; UC-1; MUM-1/m; MUM-2/m; MY01A; MY01B; MY01C; MY01D; MY01E; MY01F; MY01G; MY01H; NA17; NA88-A; Neo-PAP; NFYC/m; NGEP; N-myc; NPM; NRCAM; NSE; NUF2; NY-ESO-1; OA1; OS-9; osteocalcin; osteopontin; p53; PAGE-4; PAI-1; PAI-2; PAP; PATE; PAX3; PAX5; PD1L1; PDCD1; PDEF; PECA1; PGCB; PGFRB; Pim-1-Kinase; Pin-1; PLAC1; PMEL; PML; POTE; POTEF; PRAME; PRDX5/m; PRM2; prostein; proteinase-3; PSA; PSB9; PSCA; PSGR; PSM; PTPRC; RAB8A; RAGE-1; RARA; RASH; RASK; RASN; RGS5; RHAMM/CD168; RHOC; RSSA; RU1; RU2; RUNX1; S-100; SAGE; SART-1; SART-2; SART-3; SEPR; SERPINB5; SIA7F; SIA8A; SIAT9; SIRT2/m; SOX10; SP17; SPNXA; SPXIM3; SSX-1; SSX-2; SSX3; SSX-4; ST1A1; STAG 2; STAMP-1; STEAP-1; Survivin; Survivin-2B; SYCP1; SYT-SSX-1; SYT-SSX-2; TARP; TCRg; TF2AA; TGFbeta1; TGFR2; TGM-4; TIE2; TKTL1; TPI/m; TRGV11; TRGV9; TRPC1; TRP-p8; TSG10; TSPY1; TVCJTRGV3; TX101; tyrosinase; TYRP1; TYRP2; UPA; VEGFR1, and XAGE1; and/or a tumor neo-antigen, preferably selected from the group consisting of a single amino acid mutant peptide, a frame-shift peptide, a read-through mutation peptide, a splice site mutant peptide, and/or a senescence associated antigen.

In the fifth aspect, the invention relates to a pharmaceutical combination comprising:
(i) the combination or the single mRNA molecule according to the first aspect of this invention, the host cell according to the second aspect of this invention, the pharmaceutical composition according to the third aspect of this invention, or the vaccine according to the fourth aspect of this invention, and
(ii) a checkpoint inhibitor, optionally wherein the checkpoint inhibitor is selected from Atezolizumab, Avelumab, Cemiplimab, Dostarlimab, Durvalumab, Nivolumab, Pembrolizumab, Ipilimumab, and Relatlimab, and/or
(iii) an antagonistic antibody or an antagonistic antibody fragment or an antagonistic peptide against an immune checkpoint protein, optionally wherein the immune checkpoint protein is preferentially selected from but not restricted to any one of PD-1, cd47, cd31, PD-L1, PD-L2, BTLA, HVEM, lag3, tigit, prv, cd155, cd112, galectin-9, tim3, tim4, gitr, gitrl, tigit, Vista, cd276, cd39, cd73, CTLA4, IL10, IL35, ido, vip, IL27, and IL37.

In a preferred embodiment, the pharmaceutical combination comprises:
(i) a vaccine according to the fourth aspect of this invention, wherein the vaccine elicits a protective adaptive immune response against a cancer antigen or a tumour antigen, and
(ii) a checkpoint inhibitor, optionally wherein the checkpoint inhibitor is selected from Atezolizumab, Avelumab, Cemiplimab, Dostarlimab, Durvalumab, Nivolumab, Pembrolizumab, Ipilimumab, and Relatlimab, and/or
(iii) an antagonistic antibody or an antagonistic antibody fragment or an antagonistic peptide against an immune checkpoint protein, optionally wherein the immune checkpoint protein is preferentially selected from but not restricted to any one of PD-1, cd47, cd31, PD-L1, PD-L2, BTLA, HVEM, lag3, tigit, prv, cd155, cd112, galectin-9, tim3, tim4, gitr, gitrl, tigit, Vista, cd276, cd39, cd73, CTLA4, IL10, IL35, ido, vip, IL27, and IL37.

In the sixth aspect, the invention relates to a kit comprising the combination or the single mRNA molecule according to the first aspect of this invention, or the host cell according to the second aspect of this invention, the pharmaceutical composition according to the third aspect of this invention, the vaccine according to the fourth aspect of this invention, or the pharmaceutical combination according to the fifth aspect of the present invention, and optionally comprising a liquid vehicle for solubilising, and further optionally technical instructions providing information on administration and/or dosage of the components.

The seventh aspect of the present invention pertains to the combination or the single mRNA molecule according to the first aspect of this invention, or the host cell according to the second aspect of this invention, the pharmaceutical composition according to the third aspect of this invention, the vaccine according to the fourth aspect of this invention, the pharmaceutical combination according to the fifth aspect of the present invention, or the kit according to the sixth aspect of this invention, for use in medicine.

The eighth aspect of the present invention pertains to the combination or the single mRNA molecule according to the first aspect of this invention, or the host cell according to the second aspect of this invention, the pharmaceutical composition according to the third aspect of this invention, the vaccine according to the fourth aspect of this invention, the pharmaceutical combination according to the fifth aspect of the present invention, or the kit according to the sixth aspect of this invention, for use in a method of diagnosis, prevention, and/or treatment of an infectious, a genetic, or a proliferative disease, wherein the infectious disease is preferably a viral infection, a bacterial infection, a fungal infection, a protozoan infection, and/or a parasite infection, such as an infection caused by a corona virus, for example a COVID-19 disease, and wherein the proliferative disease is preferably cancer, more preferably a cancer selected from pancreatic cancer, bladder cancer, breast cancer, liver cancer, lung cancer, cutaneous squamous cell carcinoma, basal cell carcinoma, melanoma, uterine (endometrial) cancer, kidney cancer, skin cancer, such as Merkel cell carcinoma, colorectal cancer, esophageal cancer, gastric cancer, head and neck cancer, neuroblastoma, leukemia, sarcoma, kidney cancer, lymphoma, mesothelioma, cervical cancer, stomach cancer, and a cancer that presents with certain genetic mutations (such as MSI-H, dMMR, or TMB-H).

In another embodiment, the cancer is preferably selected from one or more of Oesophageal Cancer; Thyroid Cancer; Lung Cancer; Stomach Cancer; Pancreatic Cancer; Kidney Cancer; Cervical Cancer; Breast Cancer; Neuroendocrine Carcinoma; Endometrial Cancer; Vaginal Cancers; Blood-related Cancers; Glioma; Bone Sarcoma; Cervix Cancer; Synovial Cancer; Sarcoma; Acute Lymphoblastic Leukemia; Acute Lymphoblastic Leukemia; Acute Myeloid Leukemia; Adrenocortical Carcinoma; AIDS-Related Lymphoma; AIDS-Related Malignancies; Anal Cancer; Astrocytoma; Bile Duct Cancer, Extrahepatic; Bladder Cancer; Bone Cancer, Osteosarcoma/Malignant Fibrous Histiocytoma; Brain Stem Glioma; Cerebellar Astrocytoma; Brain Tumor, Cerebral Astrocytoma/Malignant Glioma; Ependymoma; Medulloblastoma; Brain Tumor, Supratentorial Primitive Neuroectodermal Tumors; Visual Pathway and Hypothalamic Glioma, Bronchial Adenomas/Carcinoids;; Carcinoma; Adrenocortical; Central Nervous System Lymphoma, Primary; Chronic Lymphocytic Leukemia; Chronic Myelogenous Leukemia; Chronic Myeloproliferative Disorders; Clear Cell Sarcoma of Tendon Sheaths; Colon Cancer; Cutaneous T-Cell Lymphoma; Endometrial Cancer; Ependymoma; Epithelial Cancer; Ovarian Cancer; Esophageal Cancer, Ewing's Family of Tumors; Extracranial Germ Cell Tumor; Extragonadal Germ Cell Tumor; Extrahepatic Bile Duct Cancer; Eye Cancer, Intraocular Melanoma; Eye Cancer, Retinoblastoma; Gallbladder Cancer; Gastric (Stomach) Cancer; Gastrointestinal Carcinoid Tumor; Germ Cell Tumor, Extracranial; Germ Cell Tumor, Extragonadal; Germ Cell Tumor, Ovarian; Gestational Trophoblastic Tumor; Hairy Cell Leukemia; Head and Neck Cancer; Hodgkin's Lymphoma, Hypopharyngeal Cancer; Hypothalamic and Visual Pathway Glioma; Intraocular Melanoma; Islet Cell Carcinoma (Endocrine Pancreas); Kaposi's Sarcoma; Kidney Cancer; Laryngeal Cancer, Acute Lymphoblastic; Leukemia, Acute Myeloid; Chronic Lymphocytic; Chronic Myelogenous; Hairy Cell; Lip and Oral Cavity Cancer; Lymphoblastic Leukemia; Lymphoma, AIDS- Related; Lymphoma, Central Nervous System (Primary); Lymphoma, Cutaneous T-Cell; Lymphoma, Lymphoma, Primary Central Nervous System; Macroglobulinemia, Malignant Mesothelioma; Malignant Thymoma; Medulloblastoma; Melanoma; Melanoma, Intraocular; Merkel Cell Carcinoma; Mesothelioma, Malignant; Metastatic Squamous Neck Cancer with Occult Primary; Multiple Endocrine Neoplasia Syndrome; Multiple Myeloma/Plasma Cell Neoplasm; Mycosis Fungoides; Myelodysplasia Syndromes; Myelogenous Leukemia, Chronic; Myeloid Leukemia; Myeloma, Multiple; Myeloproliferative Disorders, Chronic; Nasal Cavity and Paranasal Sinus Cancer; Nasopharyngeal Cancer; Neurofibroma; Non-Hodgkin's Lymphoma, Non-Small Cell Lung Cancer; Oral Cavity and Lip Cancer; Oropharyngeal Cancer; Osteosarcoma/Malignant Fibrous Histiocytoma of Bone; Ovarian Epithelial Cancer; Ovarian Germ Cell Tumor; Ovarian Low Malignant Potential Tumor; Pancreatic Cancer; Paranasal Sinus and Nasal Cavity Cancer; Parathyroid Cancer; Penile Cancer; Pheochromocytoma; Pineal and Supratentorial Primitive Neuroectodermal Tumors; Pituitary Tumor; Plasma Cell Neoplasm/Multiple Myeloma; Pleuropulmonary Blastoma; Primary Central Nervous System Lymphoma; Primary Liver Cancer; Prostate Cancer; Rectal Cancer; Renal Cell (Kidney) Cancer; Renal Pelvis and Ureter, Transitional Cell Cancer; Retinoblastoma; Rhabdomyosarcoma; Salivary Gland Cancer; Sarcoma, Ewing"s Family of Tumors; Sarcoma, Kaposi"s; Sarcoma (Osteosarcoma)/Malignant Fibrous Histiocytoma of Bone; Sarcoma, Rhabdomyosarcoma; Sarcoma, Soft Tissue; Sezary Syndrome; Skin Cancer (Melanoma); Skin Carcinoma, Merkel Cell; Small Cell Lung Cancer; Small Intestine Cancer; Soft Tissue Sarcoma; Squamous Neck Cancer with Occult Primary, Metastatic; Stomach (Gastric) Cancer; Supratentorial Primitive Neuroectodermal Tumors; T-Cell Lymphoma, Cutaneous; Testicular Cancer; Thymoma; Thyroid Cancer; Transitional Cell Cancer of the Renal Pelvis and Ureter; Trophoblastic Tumor, Gestational; Ureter and Renal Pelvis, Transitional Cell Cancer; Urethral Cancer; Uterine Sarcoma; Vaginal Cancer; Visual Pathway and Hypothalamic Glioma, and Vulvar Cancer.

One embodiment of the present invention pertains to the combination or the single mRNA molecule according to the first aspect of this invention, or the host cell according to the second aspect of this invention, the pharmaceutical composition according to the third aspect of this invention, the vaccine according to the fourth aspect of this invention, the pharmaceutical combination according to the fifth aspect of the present invention, or the kit according to the sixth aspect of this invention, for use according to the seventh aspect, wherein said method comprises administration of the combination or the single mRNA molecule according to the first aspect of this invention, or the host cell according to the second aspect of this invention, the pharmaceutical composition according to the third aspect of this invention, the vaccine according to the fourth aspect of this invention, the pharmaceutical combination according to the fifth aspect of the present invention, the kit according to the sixth aspect of this invention, the single vector comprising the at least one first mRNA molecule and the at least one second mRNA molecule, the two separate compositions comprising the at least one first mRNA molecule and the at least one second mRNA molecule, respectively, or the combined composition, or the single mRNA molecule, to a subject, optionally wherein the single vector comprising the at least one first mRNA molecule and the at least one second mRNA molecule, or the two separate compositions comprising the at least one first mRNA molecule and the at least one second mRNA molecule, respectively, or the combined composition, or the single mRNA molecule, are administered to the subject, further optionally wherein the two separate compositions comprising the at least one first mRNA molecule and the at least one second mRNA molecule, respectively, are administered separately, and wherein
i) the two separate compositions are administered separately at a first location site and at a second location site, wherein the first location site is within 20 cm, 17.5 cm, 15 cm, 12.5 cm, 10 cm, 7.5 cm, 5 cm, 2.5 cm, 1 cm, 0.5 cm, 0.25 cm, or 0.1 cm, of the second location site, preferably wherein the draining lymphatic system of the first location site drains to the same lymph nodes as the lymphatic system of the second location site, or wherein the first location site and the second location site are the same, and/or
ii) the two separate compositions are administered separately within a time interval of 1 hour or less, 30 minutes or less, 20 minutes or less, 15 minutes or less, 10 minutes or less, 5 minutes or less, 3 minutes or less, or 1 minute or less,
to allow a spatially and/or temporally coordinated in vivo expression of the at least one first molecule, which is a therapeutic or immunogenic protein or peptide, and the at least one second molecule, which is a protein or a peptide having the ability to modulate the immune response against the first molecule and/or the translation product of the first molecule.

A subject according to the present invention is preferably a mammal, such as a human, a mouse, a dog, a cat, a rabbit, a monkey, and so on. In a preferred embodiment, the subject is a human. In a further preferred embodiment, the subject is a human patient suffering from a disease or a human suspected of being at risk of suffering from a disease in the future.

Another embodiment of the present invention pertains to the combination or the single mRNA molecule according to the first aspect of this invention, or the host cell according to the second aspect of this invention, the pharmaceutical composition according to the third aspect of this invention, the vaccine according to the fourth aspect of this invention, the pharmaceutical combination according to the fifth aspect of the present invention, or the kit according to the sixth aspect of this invention, for use according to the seventh aspect, wherein the combination or the single mRNA molecule according to the first aspect of this invention, wherein the treatment further comprises radiation therapy and/or surgery.

Another embodiment of the present invention pertains to the combination or the single mRNA molecule according to the first aspect of this invention, or the host cell according to the second aspect of this invention, the pharmaceutical composition according to the third aspect of this invention, the vaccine according to the fourth aspect of this invention, the pharmaceutical combination according to the fifth aspect of the present invention, or the kit according to the sixth aspect of this invention, for use according to the seventh aspect, wherein the combination or the single mRNA molecule according to the first aspect of this invention, or the host cell according to the second aspect of this invention, the pharmaceutical composition according to the third aspect of this invention, the vaccine according to the fourth aspect of this invention, the pharmaceutical combination according to the fifth aspect of the present invention, or the kit according to the sixth aspect of this invention, the single vector comprising the at least one first mRNA molecule and the at least one second mRNA molecule, the two separate compositions comprising the at least one first mRNA molecule and the at least one second mRNA molecule, respectively, or the combined composition, or the single mRNA molecule, are administered to the subject by intramuscular, subcutaneous, intradermal, intra-peritoneal, intravenous, and/or intra-pleural injection, wherein, preferably, the two separate compositions comprising the at least one first mRNA molecule and the at least one second mRNA molecule, respectively, are administered by the same route.

Another aspect of the present invention, which can be combined with any other specifically preferred embodiment and/or aspect of the present invention, pertains to a method of diagnosis, prevention, and/or treatment of an infectious, a genetic, or a proliferative disease, comprising the administration of the combination or the single mRNA molecule according to the first aspect of this invention, or the host cell according to the second aspect of this invention, the pharmaceutical composition according to the third aspect of this invention, the vaccine according to the fourth aspect of this invention, the pharmaceutical combination according to the fifth aspect of the present invention, or the kit according to the sixth aspect of this invention to a subject, wherein the infectious disease is preferably a viral infection, a bacterial infection, a fungal infection, a protozoan infection, and/or a parasite infection, such as an infection caused by a corona virus, for example a COVID-19 disease, and wherein the proliferative disease is preferably cancer, more preferably a cancer selected from pancreatic cancer, bladder cancer, breast cancer, liver cancer, lung cancer, cutaneous squamous cell carcinoma, basal cell carcinoma, melanoma, uterine (endometrial) cancer, kidney cancer, skin cancer, such as Merkel cell carcinoma, colorectal cancer, esophageal cancer, gastric cancer, head and neck cancer, neuroblastoma, leukemia, sarcoma, kidney cancer, lymphoma, mesothelioma, cervical cancer, stomach cancer, and a cancer that presents with certain genetic mutations (such as MSI-H, dMMR, or TMB-H).

The subject is preferably a mammal, such as a human, a mouse, a dog, a cat, a rabbit, or a monkey. In a preferred embodiment, the subject is a human. In a further preferred embodiment, the subject is a human patient suffering from a disease or a human suspected of being at risk of suffering from a disease in the future.

A further aspect of the present invention, which can be combined with any other specifically preferred embodiment and/or aspects of the present invention, pertains to the combination or the single mRNA molecule according to the first aspect of this invention, or the host cell according to the second aspect of this invention, the pharmaceutical composition according to the third aspect of this invention, the vaccine according to the fourth aspect of this invention, the pharmaceutical combination according to the fifth aspect of the present invention, or the kit according to the sixth aspect of this invention, in the manufacture of a medicament for the treatment of an infectious, a genetic, or a proliferative disease, wherein the infectious disease is preferably a viral infection, a bacterial infection, a fungal infection, a protozoan infection, and/or a parasite infection, such as an infection caused by a corona virus, for example a COVID-19 disease, and wherein the proliferative disease is preferably cancer, more preferably a cancer selected from pancreatic cancer, bladder cancer, breast cancer, liver cancer, lung cancer, cutaneous squamous cell carcinoma, basal cell carcinoma, melanoma, uterine (endometrial) cancer, kidney cancer, skin cancer, such as Merkel cell carcinoma, colorectal cancer, esophageal cancer, gastric cancer, head and neck cancer, neuroblastoma, leukemia, sarcoma, kidney cancer, lymphoma, mesothelioma, cervical cancer, stomach cancer, and a cancer that presents with certain genetic mutations (such as MSI-H, dMMR, or TMB-H).

Another aspect of the present invention pertains to the combination or the single mRNA molecule according to the first aspect of this invention, or the host cell according to the second aspect of this invention, the pharmaceutical composition according to the third aspect of this invention, the vaccine according to the fourth aspect of this invention, the pharmaceutical combination according to the fifth aspect of the present invention, or the kit according to the sixth aspect of this invention, for use in the diagnosis, prevention, and/or treatment of an infectious, a genetic, or a proliferative disease, wherein the combination or the single mRNA molecule, the host cell, the pharmaceutical composition, the vaccine, the pharmaceutical combination, or the kit, stimulates neutralizing antibodies and/or other protective antibodies, and/or T cells against infectious disease antigens and/or cancer antigens in the subject.

Another aspect of the present invention pertains to a method of manufacturing the combination or the single mRNA molecule according to the first aspect of this invention, or the host cell according to the second aspect of this invention, the pharmaceutical composition according to the third aspect of this invention, the vaccine according to the fourth aspect of this invention, the pharmaceutical combination according to the fifth aspect of the present invention, or the kit according to the sixth aspect of this invention.

The terms "of the [present] invention", "in accordance with the invention", "according to the invention" and the like, as used herein are intended to refer to all aspects and embodiments of the invention described and/or claimed herein.

As used herein, the term "comprising" is to be construed as encompassing both "including" and "consisting of', both meanings being specifically intended, and hence individually disclosed embodiments in accordance with the present invention. Where used herein, "and/or" is to be taken as specific disclosure of each of the two specified features or components with or without the other. For example, "A and/or B" is to be taken as specific disclosure of each of (i) A, (ii) B and (iii) A and B, just as if each is set out individually herein. In the context of the present invention, the terms "about" and "approximately" denote an interval of accuracy that the person skilled in the art will understand to still ensure the technical effect of the feature in question. The term typically indicates deviation from the indicated numerical value by ±20%, ±15%, ±10%, and for example ±5%. As will be appreciated by the person of ordinary skill, the specific such deviation for a numerical value for a given technical effect will depend on the nature of the technical effect. For example, a natural or biological technical effect may generally have a larger such deviation than one for a man-made or engineering technical effect. As will be appreciated by the person of ordinary skill, the specific such deviation for a numerical value for a given technical effect will depend on the nature of the technical effect. For example, a natural or biological technical effect may generally have a larger such deviation than one for a man-made or engineering technical effect. Where an indefinite or definite article is used when referring to a singular noun, e.g. "a", "an" or "the", this includes a plural of that noun unless something else is specifically stated.

It is to be understood that application of the teachings of the present invention to a specific problem or environment, and the inclusion of variations of the present invention or additional features thereto (such as further aspects and embodiments), will be within the capabilities of one having ordinary skill in the art in light of the teachings contained herein.

Unless context dictates otherwise, the descriptions and definitions of the features set out above are not limited to any particular aspect or embodiment of the invention and apply equally to all aspects and embodiments which are described.

All references, patents, and publications cited herein are hereby incorporated by reference in their entirety.

### BRIEF DESCRIPTION OF THE FIGURES

The figures show:

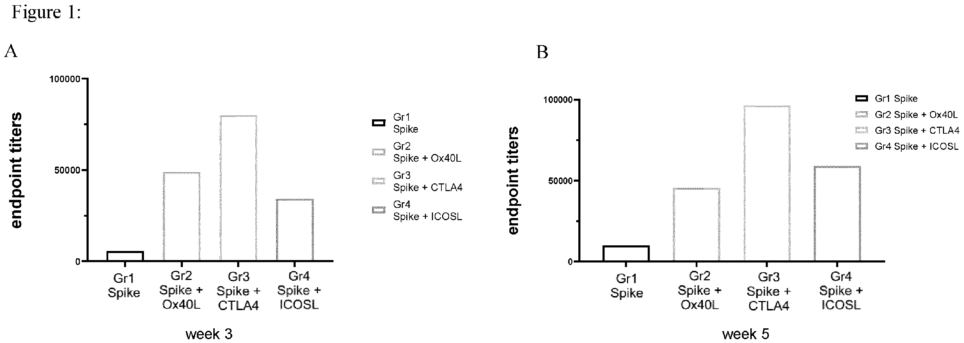

**Figure 1** **shows the improvement of humoral response induced by a COVID mRNA vaccine using a combination composed of the mRNA vaccine and mRNA encoding modulating peptides.** Groups of mice were immunized intramuscularly with: 7µg of mRNA encoding for the SARS-Cov2 Spike protein formulated in lipo-nanoparticles (LNP) (Gr1 - Spike); a mixture (combination) including 7µg of mRNA encoding for the SARS-Cov2 Spike protein formulated in LNP and 21µg of mRNA encoding for the Ox40 ligand protein in LNP (Gr2 - Spike + Ox40L); a mixture (combination) including 7µg of mRNA encoding for the SARS-Cov2 Spike protein in LNP and 7µg of mRNA encoding for an antibody against CTLA4 in LNP (Gr3 - Spike + CTLA4), and a mixture (combination) including 7µg of mRNA encoding for the SARS-Cov2 Spike protein in LNP and 7µg of mRNA encoding for the ICOS ligand protein in LNP (Gr4 - Spike + ICOSL). Fig. 1 A: Sera from LNP mRNA immunized mice were tested by ELISA for their antibody titers against the SARS-Cov2 Receptor Binding Domain (RBD) at three weeks after immunization. Fig. 1 B: Sera from LNP mRNA immunized mice were tested by ELISA for their antibody titers against the SARS-Cov2 Receptor Binding Domain (RBD) at five weeks after immunization.

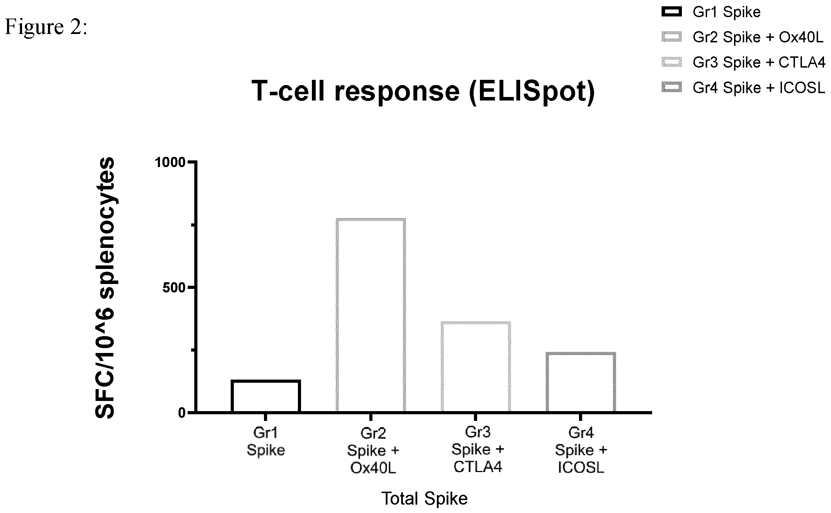

**Figure 2** **shows the improvement of cellular response induced by a COVID mRNA vaccine using a combination composed of the mRNA vaccine and mRNA encoding modulator peptides.** Groups of mice were immunized intramuscularly with: 7µg of mRNA encoding for the SARS-Cov2 Spike protein formulated in lipo-nanoparticles (LNP) (Gr1 - Spike); a mixture (combination) including 7µg of mRNA encoding for the SARS-Cov2 Spike protein formulated in LNP and 21µg of mRNA encoding for the Ox40 ligand protein in LNP (Gr2 - Spike + Ox40L); a mixture (combination) including 7µg of mRNA encoding for the SARS-Cov2 Spike protein in LNP and 21µg of mRNA encoding for an antibody against CTLA4 in LNP (Gr3 - Spike + CTLA4), and a mixture (combination) including 7µg of mRNA encoding for the SARS-Cov2 Spike protein in LNP and 21µg ofmRNA encoding for the ICOS ligand protein in LNP (Gr4 - Spike + ICOSL). T-cell responses of immunized mice were tested by *ex vivo* IFNgamma ELIspot using synthetic peptides reproducing the amino acid sequence of the Spike protein.

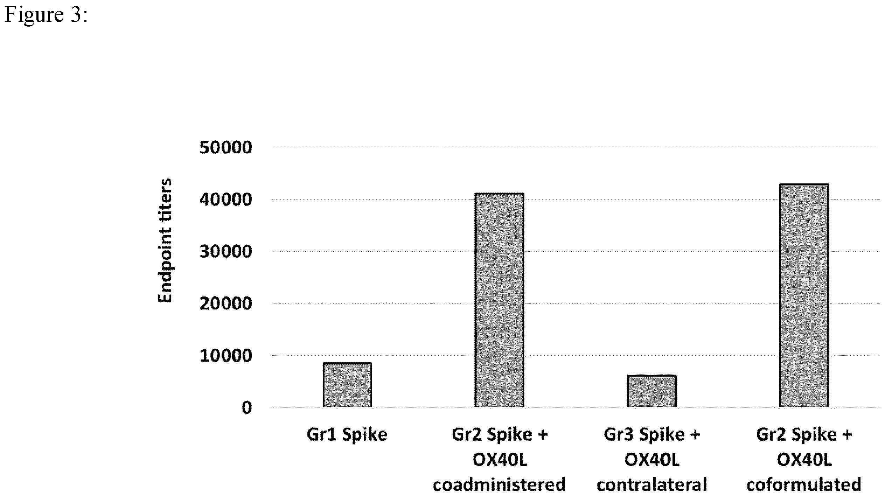

**Figure 3** **shows that the activity of the immunomodulating RNA requires co-formulation or co-administration with the therapeutic RNA (assessed by B cell responses).** A control group of mice was immunized with LNP-Spike only (Gr1 - Spike). In a second group of mice, LNP containing the mRNA encoding for the SARS-Cov2 Spike protein (LNP-Spike) and LNP containing the mRNA encoding for the Ox40 ligand protein (LNP-Ox40L) were prepared separately and then and mixed prior to administration in the same mouse quadricep (Gr2 - Spike + Ox40L co-administered). In a third group of mice, the same amounts of LNP-Spike and LNP-Ox40L were injected contralaterally in the left and right quadriceps, respectively (Gr3 Spike & OX40L contralateral). A fourth group of mice was immunized with the same amounts of mRNA encoding for the Spike protein and mRNA encoding for the Ox40 ligand protein co-formulated into the same LNP (Gr3 Spike & OX40L co-formulated). B cell responses were assessed in sera from immunized mice five weeks after immunization and were tested by ELISA for antibody titers against the SARS-Cov2 Receptor Binding Domain (RBD).

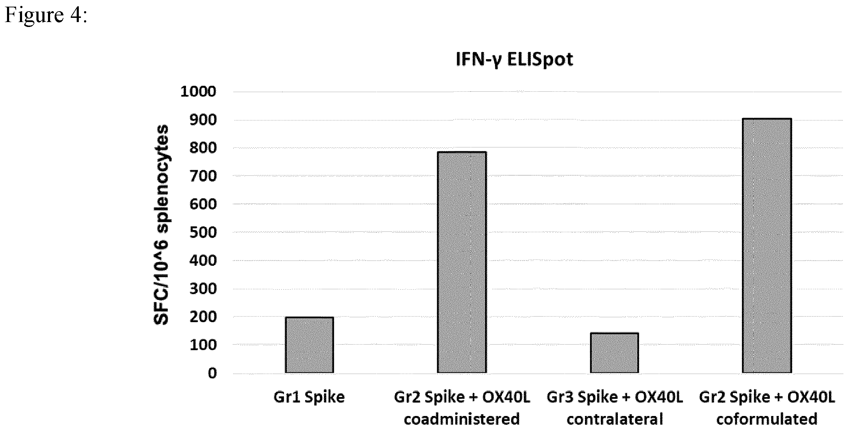

**Figure 4** **shows that the activity of the immunomodulating RNA requires co-formulation or co-administration with the therapeutic RNA (assessed by T cell responses).** A control group of mice was immunized with LNP-Spike only (Gr1 - Spike). In a second group of mice, LNP containing the mRNA encoding for the SARS-Cov2 Spike protein (LNP-Spike) and LNP containing the mRNA encoding for the Ox40 ligand protein (LNP-Ox40L) were prepared separately and then and mixed prior to administration in the same mouse quadricep (Gr2 - Spike + Ox40L co-administered). In a third group of mice, the same amounts of LNP-Spike and LNP-Ox40L were injected contralaterally in the left and right quadriceps, respectively (Gr3 Spike & OX40L contralateral). A fourth group of mice was immunized with the same amounts of mRNA encoding for the Spike protein and mRNA encoding for the Ox40 ligand protein co-formulated into the same LNP (Gr3 Spike & OX40L co-formulated). T-cell responses were measured after five weeks from the immunization by *ex vivo* IFNgamma ELIspot using Spike peptides.

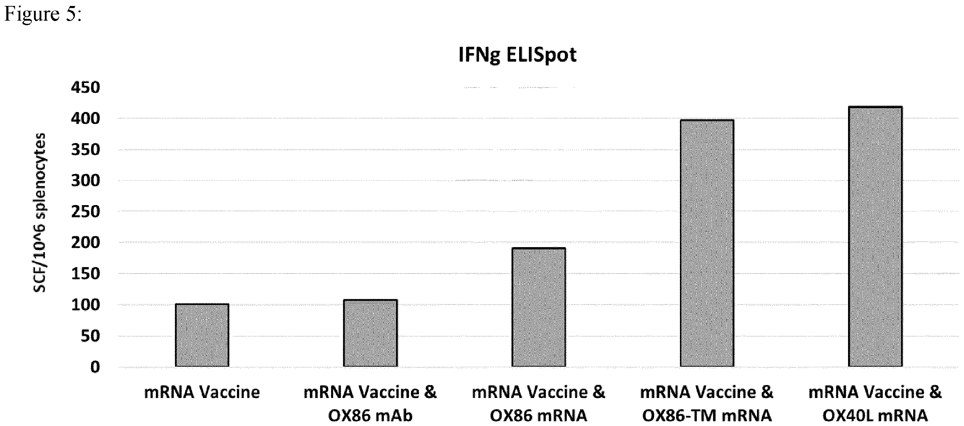

**Figure 5** **shows that mRNA encoding positive immunomodulators of the cell-bound receptors or ligands category (native protein or agonistic antibody or agonistic peptide) need to be cell bound for optimal activity (assessed by T cell responses).** A control group of mice was immunized with the Spike RNA vaccine only (mRNA Vaccine). In a second group of mice, the Spike RNA vaccine was administered with an OX40 agonistic antibody (OX86) in its proteinaceous form (mRNA Vaccine & OX86 mAb). A third group received the same mRNA/LNP vaccine together with the mRNA/LNP encoding the OX86 antibody (mRNA Vaccine & OX86 mRNA). A fourth group received the same mRNA/LNP vaccine together with the mRNA/LNP encoding a membrane anchored version of the OX86 antibody (TM-OX86)( mRNA Vaccine & OX86-TM mRNA). The last group of mice received the same mRNA/LNP vaccine together with the mRNA/LNP encoding the native OX40 ligand (mRNA Vaccine & OX40L mRNA). The anti-Spike cellular immunity (T-cell response) was measured by *ex vivo* IFNgamma ELIspot after five weeks from the immunization.

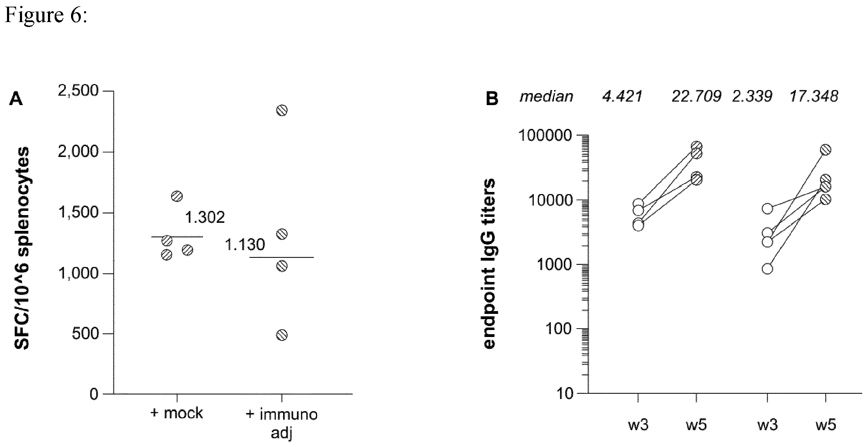

**Figure 6** **shows that the activity of nucleic acid encoded immunomodulators depends on the vector/nucleic acid type and cannot be predicted (DNA encoded OX40L does not improve the immunogenicity of co-delivered DNA vaccine).** A first control group of mice was immunized intramuscularly by *in vivo* electroporation with a DNA plasmid encoding the SARS-Cov2 Spike protein mixed with a mock non-coding DNA plasmid. A second group of mice was similarly immunized by *in vivo* electroporation with a DNA plasmid encoding the SARS-Cov2 Spike protein mixed with a plasmid encoding OX40L. T cell response was evaluated by *ex vivo* IFNgamma ELIspot (A); end-point anti Spike IgG titers were measured by ELISA (B).

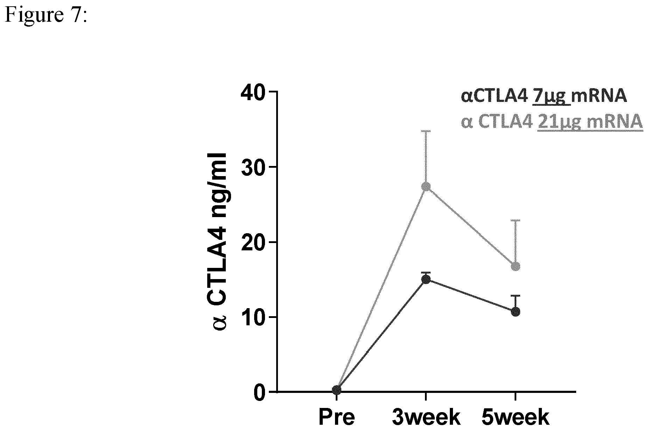

**Figure 7** **shows the kinetic evaluation of RNA-encoded, LNP-formulated aCTLA4 antibody concentration from sera of immunized mice.** Mice received the Spike RNA vaccine with two different doses of mRNA encoding for the antibody against CTLA4 in LNP as reported in figure 1 and 2. Sera were collected from mice before intramuscular administration of RNAs and at week 3 and week 5 post injection. Anti CTLA4 antibody was measured by ELISA assay using CTLA4 coated plates as capture antigen.

### EXAMPLES

Certain aspects and embodiments of the invention will now be illustrated by way of example and with reference to the description, figures and tables set out herein. Such examples of the methods, uses and other aspects of the present invention are representative only, and should not be taken to limit the scope of the present invention to only such representative examples.

The examples show:

### Example 1: Improvement of humoral response induced by a COVID mRNA vaccine using a combination composed of the mRNA vaccine and mRNA encoding modulating peptides

To show the adjuvant effect of modulating RNA on the immunological activity of an mRNA vaccine, the inventors immunized groups of mice with the mRNA vaccine alone or with the mRNA vaccine mixed with the mRNA encoding peptides with immunomodulating activity.

Groups of mice were immunized intramuscularly with: a) 7µg of mRNA encoding for the SARS-Cov2 Spike protein formulated in lipo-nanoparticles (LNP) (Gr1 - Spike); b) a mixture (combination) including 7µg of mRNA encoding for the SARS-Cov2 Spike protein formulated in LNP and 21 µg of mRNA encoding for the Ox40 ligand protein in LNP (Gr2 - Spike + Ox40L); c) a mixture (combination) including 7µg of mRNA encoding for the SARS-Cov2 Spike protein in LNP and 7µg of mRNA encoding for an antibody against the anti-CTLA4 in LNP (Gr3 - Spike + CTLA4); d) a mixture (combination) including 7µg of mRNA encoding for the SARS-Cov2 Spike protein in LNP and 7µg of mRNA encoding for the ICOS ligand protein in LNP (Gr4 - Spike + ICOSL).

All the mRNAs were transcribed in vitro using N1mPseudoU and synthetic CAP1. LNP were composed of ionizable cat ionic lipid, neutral lipid, cholesterol and PEGylated lipid in the appropriate ratio. Sera from immunized mice were tested by ELISA for their antibody titers against the SARS-Cov2 Receptor Binding Domain (RBD) at three and five weeks after immunization (Figure 1).

The results shown in Figure 1 indicate that co-administration of the different immunomodulator mRNAs increase anti-RBD antibody titers at both time points, indicating that the inventors' approach improves both potency and durability of vaccine induced B-cell adaptive immunity.

### Example 2: Improvement of cellular response induced by a COVID mRNA vaccine using a combination composed of the mRNA vaccine and mRNA encoding modulator peptides

To show that the adjuvant effect of the modulating RNA on the immunological activity of an mRNA vaccine, acts also on the vaccine induced T cell immunity the inventors immunized groups of mice with the Spike mRNA vaccine alone or with the Spike mRNA vaccine mixed with the mRNA encoding peptides with immunomodulating activity and measured the anti-Spike cellular immunity by *ex vivo* IFNgamma ELIspot after five weeks from the immunization.

Groups of mice were immunized intramuscularly with: a) 7µg of mRNA encoding for the SARS-Cov2 Spike protein formulated in lipo-nanoparticles (LNP) (Gr1 - Spike); b) a mixture (combination) including 7µg of mRNA encoding for the SARS-Cov2 Spike protein formulated in LNP and 21 µg of mRNA encoding for the Ox40 ligand protein in LNP (Gr2 - Spike + Ox40L); c) a mixture (combination) including 7µg of mRNA encoding for the SARS-Cov2 Spike protein in LNP and 21µg of mRNA encoding for an antibody against the anti-CTLA4 in LNP (Gr3 - Spike + CTLA4); d) a mixture (combination) including 7µg of mRNA encoding for the SARS-Cov2 Spike protein in LNP and 21µg of mRNA encoding for the ICOS ligand protein in LNP (Gr4 - Spike + ICOSL).

All the mRNAs were transcribed *in vitro* using N1mPseudoU and synthetic CAP1. LNP were composed of ionizable cat ionic lipid, neutral lipid, cholesterol and PEGylated lipid in the appropriate ratio.

Splenocytes from immunized mice were tested by *ex vivo* IFNgamma ELIspot using synthetic peptides reproducing the amino acid sequence of the Spike protein (according to SEQ ID No: 1).

The results shown in Figure 2 indicate that co-administration of the different immunomodulator mRNAs increase anti-Spike T cell immune responses.

### Example 3: The activity of the immunomodulating RNA requires co-formulation or co-administration with the therapeutic RNA

To demonstrate that the increase of the adaptive immunity requires the coordinated expression of the therapeutic and the modulating RNAs, the inventors immunized mice with different modalities of administration. In a first group of mice, a control group was immunized with LNP-Spike only (Gr1 - Spike). In a second group of mice, LNP containing the mRNA encoding for the SARS-Cov2 Spike protein (LNP-Spike) and LNP containing the mRNA encoding for the Ox40 ligand protein (LNP-Ox40L) were prepared separately and then and mixed prior to administration in the same mouse quadricep (Gr2 - Spike + Ox40L co-administered). In a third group of mice, the same amounts of LNP-Spike and LNP-Ox40L were injected contralaterally in the left and right quadriceps, respectively (Gr3 Spike & OX40L contralateral). A fourth group of mice was immunized with the same amounts of mRNA encoding for the Spike protein and mRNA encoding for the Ox40 ligand protein co-formulated into the same LNP (Gr3 Spike & OX40L co-formulated).

B cell responses were assessed in sera from immunized mice five weeks after immunization and were tested by ELISA for antibody titers against the SARS-Cov2 Receptor Binding Domain (RBD) (Figure 3). As expected, antibody titers were significantly improved when mRNA encoding Spike was co-administered with mRNA encoding OX40L. Contralateral administration of therapeutic and modulatory mRNA completely abrogated the enhancement (Figure 3).

The inventors performed a further experiment to assess the immunomodulating activity of the different modalities of administration on the vaccine induced T-cell responses. In a first group of mice, a control group was immunized with LNP-Spike only (Gr1 - Spike). In a second group of mice, LNP containing the mRNA encoding for the SARS-Cov2 Spike protein (LNP-Spike) and LNP containing the mRNA encoding for the Ox40 ligand protein (LNP-Ox40L) were prepared separately and then and mixed prior to administration in the same mouse quadricep (Gr2 - Spike + Ox40L co-administered). In a third group of mice, the same amounts of LNP-Spike and LNP-Ox40L were injected contralaterally in the left and right quadriceps, respectively (Gr3 Spike & OX40L contralateral). A fourth group of mice was immunized with the same amounts of mRNA encoding for the Spike protein and mRNA encoding for the Ox40 ligand protein co-formulated into the same LNP (Gr3 Spike & OX40L co-formulated). Then, T-cell responses were measured after five weeks from the immunization by *ex vivo* IFNgamma ELIspot using Spike peptides. As for the B cell response, also T cell reactivity resulted enhanced when therapeutic mRNA was co-administered with the modulatory mRNA. Contralateral administration of therapeutic and modulatory mRNA completely abrogated the enhancement (Figure 4).

These results demonstrate the need to combine the therapeutic and modulatory RNAs to exert the desired effect.

### Example 4: mRNA encoding positive immunomodulators of the cell-bound receptors or ligands category (native protein or agonistic antibody or agonistic peptide) need to be cell bound for optimal activity

To further investigate the mechanism of action of mRNA encoded immunomodulators belonging to the category of cell-bound receptors or ligands (i.e., OX40 agonist), the inventors immunized groups of mice with the Spike mRNA/LNP vaccine alone or with the Spike mRNA/LNP vaccine mixed with mRNA/LNP encoding different format of OX40 agonists.

In a first group of mice, a control group was immunized with the Spike RNA vaccine only. In a second group of mice, the Spike RNA vaccine was administered with an OX40 agonistic antibody (OX86) in its proteinaceous form. A third group received the same mRNA/LNP vaccine together with the mRNA/LNP encoding the OX86 antibody. A fourth group received the same mRNA/LNP vaccine together with the mRNA/LNP encoding a membrane anchored version of the OX86 antibody (TM-OX86) obtained by adding to the C terminus of the heavy chain the transmembrane domain of the precursor IgG. The last group of mice received the same mRNA/LNP vaccine together with the mRNA/LNP encoding the native OX40 ligand. The anti-Spike cellular immunity was measured by *ex vivo* IFNgamma ELIspot after five weeks from the immunization.

As shown in figure 5, the co-administered OX86 agonistic antibody in its proteinaceous form did not enhance the vaccine immune response. On the contrary, the mRNA/LNP encoded transmembrane version of OX86 (TM-OX86) improved vaccine immunogenicity to a comparable extent of the mRNA/LNP encoded membrane anchored native ligand OX40L. The mRNA/LNP encoded soluble OX86 antibody improved vaccine immunogenicity to a lower extent.

### Example 5: Activity of nucleic acid encoded immunomodulators depends on the vector/nucleic acid type and cannot be predicted (DNA encoded OX40L does not improve the immunogenicity of co-delivered DNA vaccine)

To assess if the co-delivery of a nucleic acid based vaccine in combination with a nucleic acid based immunomodulator agent leads to improved immunogenicity independently from the type of vector or nucleic acid, the inventors tested the ability of a DNA encoded OX40L to improve immunogenicity of a DNA encoded Spike vaccine.

As shown in figure 6, co-delivery of the DNA encoded OX40L did not improve either B or T cell vaccine induced immune responses.

### Example 6: The activity of encoded immunomodulator does not rely on systemic delivery of the payload.

To assess if the activity of immunomodulator is based on local (co-injection site with the therapeutic mRNA) or systemic spread of the molecule, the inventors tested the concentration of the secreted anti-CTLA4 antibody used as immunomodulator reported in examples 1 and 2 (Figure 1 and figure 2). As shown in Figure 7, the mRNA encoded LNP formulated anti-CTLA4 antibody was administered intramuscularly at two different doses, where very low systemic concentrations were measured. These concentrations (<30ng/ml) are far away from those needed to elicit typical systemic immune response when administered as recombinant protein (>1000 ng/ml).

## Claims

1. A combination comprising two or more mRNA molecules, or a single mRNA molecule encoding a first molecule, which is a therapeutic or immunogenic protein or peptide, and a second molecule, which is a protein or a peptide having the ability to modulate an immune response against the first molecule and/or the translation product of the first molecule, wherein the combination comprises:
(i) at least one first mRNA molecule encoding a first molecule, which is a therapeutic or immunogenic protein or peptide, and
(ii) at least one second mRNA molecule encoding a second molecule, which is a protein or a peptide having the ability to modulate an immune response against the first molecule and/or the translation product of the first molecule,
wherein the at least one first mRNA molecule and the at least one second mRNA molecule are co-formulated into a single vector, or wherein the at least one first mRNA molecule and the at least one second mRNA molecule are formulated into two separate compositions, optionally wherein the two separate compositions are mixed prior to administration to form a combined composition, or wherein the first molecule and the second molecule are translated from the single mRNA molecule, for example by an internal ribosomal entry site (IRES) or by a self-cleaving peptide (such as a 2A peptide), optionally wherein the single mRNA molecule is a bicystronic or a polycystronic mRNA molecule,
wherein the at least one second mRNA molecule or the part of the single mRNA molecule encoding the second molecule is selected from the group consisting of an mRNA molecule encoding a cell-bound (or membrane-anchored) agonistic antibody or agonistic antibody fragment, or an agonistic peptide against a cell-bound (or membrane-anchored) receptor or ligand, an mRNA molecule encoding for a cell-bound (or membrane-anchored) receptor or ligand, an mRNA molecule encoding for a chemokine or a cytokine, an mRNA molecule encoding for an antagonistic antibody or an antagonistic antibody fragment against an immune checkpoint protein, an mRNA molecule encoding for an immune checkpoint inhibitor, an mRNA molecule encoding for an immune checkpoint, and an mRNA molecule encoding for a transcription factor.

2. The combination or the single mRNA molecule according to claim 1, wherein the at least one second mRNA molecule or the part of the single mRNA molecule encoding the second molecule is encoding a protein with a positive immunomodulating activity, such as a protein with the ability to increase the potency and/or the quality and/or the longevity of an immune response, or wherein the at least one second mRNA molecule or the part of the single mRNA molecule encoding the second molecule is encoding a protein with a negative immunomodulating activity, such as a protein with the ability to decrease the potency and/or the quality and/or the longevity of an immune response.

3. The combination or the single mRNA molecule according to claim 1 or 2, wherein the single vector comprising the at least one first mRNA molecule and the at least one second mRNA molecule, or the single mRNA molecule, is comprised in a nanoparticle, which is preferably a lipo-nanoparticle (LNP) or a non-lipid based nanoparticle (such as a nanoparticle composed of polymers), and/or wherein the two separate compositions comprising the at least one first mRNA molecule and the at least one second mRNA molecule, respectively, are selected from nanoparticles, synthetic particles for *in vivo* transduction, extracellular vesicles, and viral vectors, preferably wherein the nanoparticles are LNPs or non-lipid based nanoparticles (such as nanoparticles composed of polymers), wherein the LNPs are preferably composed of ionizable cationic lipid, neutral lipid (helper lipid), cholesterol, and PEGylated lipid, optionally wherein the molar lipid ratio (%) of ionizable cationic lipid:neutral lipid:cholesterol:PEG-ylated lipid in the composition is 50: 10:38.5: 1.5 or 46.3:9.4:42.7: 1.6, and wherein the N/P ratio is preferably, but not limited to, 3, 6, 4, or 8, wherein N is ionizable cationic lipid (nitrogen) and P is nucleotide (phosphate), and/or wherein the single vector comprising the at least one first mRNA molecule and the at least one second mRNA molecule, or each of the two separate compositions comprising the at least one first mRNA molecule and the at least one second mRNA molecule, or the single mRNA molecule, is a self-replicating RNA or a circular RNA, and/or wherein the single vector comprising the at least one first mRNA molecule and the at least one second mRNA molecule, or each of the two separate compositions comprising the at least one first mRNA molecule and the at least one second mRNA molecule, or the single mRNA molecule, is a viral vector.

4. The combination or the single mRNA molecule according to any one of claims 1 to 3, wherein the at least one second mRNA molecule or the part of the single mRNA molecule encoding the second molecule is encoding:
i) a chemokine or a cytokine (either soluble or cell-bound or membrane-anchored) preferentially selected from but not restricted to the following list: Leptin, IL21, Ccl19, Ada, IL4, Ccl5, Cxcl9, Cxcl10, IL7, Ccl20, Ccl21a, Cxcl12, Ccl2, HMGB1, Ccl3, IL2, Efnb2, Ifng, IL12a, IL12b, IL1a, IL1b, IL36, TL1A, Cxcl13, Cxcl16, Ifnb1, IL13, IL17a, IL18, IL23a, IL6, Nlrp3, Ccl12, Ccl6, Ccl7, Ccl8, Chemerin, Csf2, Cxcl11, IL5, Ccl11, Ccl24, Ccl26, Ccl4, Csf1, IL11, Ccl17, Ccl22, Ccl25,Ccl9, Cxcl1, Cxcl14, Cxcl15, Cxcl2, Cxcl3, Cxcl5, IL22, IL3, Ccl27a, Ccl28, IL-12 single chain, IFNα, GM-CSF, IL15, IL-15sushi, and IL-15-receptor α chain chimeric protein, or
ii) a cell-bound (or membrane-anchored) ligand preferentially selected from but not restricted to the following list: LIGHT-Tnfsf14, OX40L-Tnfsf4, GITRL-Tnfsf18, 41BBL-Tnfsf9, Cd401g, Cd70, ICOSL, Cd80, Icam1, Flt3l, CALR, Cd86, APRIL-Tnfsf13, CD166, LFA3, CD30L-Tnfsf8, Cd48, Cd74, Cd320, Cd83, Dpp4, Cd1d1, Cdld2, Clec4n, Clec5a, ITGAL, Slc11a1, Vcam1, CAV1, Cd14, PLXNB2, and TWEAK-Tnfsf12, or
iii) an agonistic antibody (preferably cell-bound or membrane-anchored) or an agonistic antibody fragment (preferably cell-bound or membrane-anchored) or an agonistic peptide (preferably cell-bound or membrane-anchored) having the same target of the molecules included in i) and/or ii).

5. The combination or the single mRNA molecule according to any one of claims 1 to 3, wherein the at least one second mRNA molecule or the part of the single mRNA molecule encoding the second molecule is encoding an antagonistic antibody or an antagonistic antibody fragment or an antagonistic peptide against an immune checkpoint protein, or an mRNA molecule encoding for an immune checkpoint inhibitor, or an mRNA molecule encoding for an immune checkpoint, and wherein the immune checkpoint protein is preferentially selected from but not restricted to any one of PD-1, cd47, cd31, PD-L1, PD-L2, BTLA, HVEM, lag3, tigit, prv, cd155, cd112, galectin-9, tim3, tim4, gitr, gitrl, tigit, Vista, cd276, cd39, cd73, CTLA4, IL10, IL35, ido, vip, IL27, and IL37.

6. The combination or the single mRNA molecule according to any one of claims 1 to 3, wherein the at least one second mRNA molecule or the part of the single mRNA molecule encoding the second molecule is encoding a transcription factor, and wherein the transcription factor is preferentially selected from but not restricted to any one of Tcf1, TFEB, TFE3, NLRC5, CIITA, Lef1, Bcl11b, Gata3, Snai3, Ets1, IRF3, IRF7, Zbtb1, irf4, stat5b, runx3, bel11a, foxo1, ikzf3, ets1, Tcf4, pax5, Bhlhe41, irf3, irf5, irf8, eomes, Stat1, Stat3, StatS, Cebpd, and Cebpa.

7. The combination or the single mRNA molecule according to any one of claims 1 to 3, or 5, wherein the at least one second mRNA molecule or the part of the single mRNA molecule encoding the second molecule is encoding for an antagonistic antibody or an antagonistic antibody fragment against an immune checkpoint protein or encoding an immune checkpoint inhibitor, and wherein:
(i) the antagonistic antibody or the antagonistic antibody fragment is targeting the PD-1/PD-L1 pathway, preferably wherein the mRNA molecule is encoding for an antibody selected from Atezolizumab, Avelumab, Cemiplimab, Dostarlimab, Durvalumab, Nivolumab, and Pembrolizumab;
(ii) the antagonistic antibody or the antagonistic antibody fragment is targeting the CTLA-4 pathway, preferably wherein the mRNA molecule is encoding for the antibody Ipilimumab, and/or
(iii) the antagonistic antibody or the antagonistic antibody fragment is targeting the LAG-3 pathway, preferably wherein the mRNA molecule is encoding for the antibody Relatlimab.

8. The combination or the single mRNA molecule according to any one of claims 1 to 4, wherein the at least one second mRNA molecule or the part of the single mRNA molecule encoding the second molecule is encoding a chemokine or a cytokine, and wherein:
(i) the cytokine targets the IL-2/IL-2R pathway, preferably wherein the mRNA molecule is encoding for the cytokine Aldesleukin;
(ii) the mRNA molecule is encoding for an immunomodulatory cytokine, such as Granulocytemacrophage colony-stimulating factor (GM-CSF), and/or
(iii) the cytokine targets the IFNAR1/2 pathway.

9. The combination or the single mRNA molecule according to any one of claims 1 to 8, wherein the at least one first mRNA molecule or the part of the single mRNA molecule encoding the first molecule is encoding a therapeutic or immunogenic protein or peptide, which is preferably a therapeutic or immunogenic protein or peptide against an infectious disease antigen, such as an antigen selected from the group consisting of bacterial antigens, for example Gram-positive bacterial antigens or Gram-negative bacterial antigens, protozoan antigens, viral antigens, fungal antigens, and parasite antigens, or a cancer antigen, tumour antigen, and/or tumor neo-antigen, preferably selected from the group consisting of a single amino acid mutant peptide, a frame-shift peptide, a read-through mutation peptide, a splice site mutant peptide, and/or a senescence associated antigen, or wherein the at least one first mRNA molecule or the part of the single mRNA molecule encoding the first molecule is encoding a CAS protein, such as a CAS9 protein, or wherein the at least one first mRNA molecule or the part of the single mRNA molecule encoding the first molecule is encoding a therapeutic protein capable of correcting a disease due to a lack of a resident gene encoding the therapeutic protein or a mutation in the resident gene encoding the therapeutic protein or an inactivation of the resident gene encoding the therapeutic protein.

10. The combination or the single mRNA molecule according to any one of claims 1 to 9, wherein the at least one first mRNA molecule and the at least one second mRNA molecule, or the single mRNA molecule, is a synthetic and/or modified mRNA molecule, preferably wherein chemically modified nucleosides are incorporated, preferentially at a uracil nucleobase and/or at a cytidine nucleobase, in said at least one first mRNA molecule and said at least one second mRNA molecule, or in said single mRNA molecule, wherein every uracil nucleobase and/or every cytidine nucleobase in said at least one first mRNA molecule and said at least one second mRNA molecule, or in said single mRNA molecule, or a given percentage of uracil nucleobases and/or cytidine nucleobases in said at least one first mRNA molecule and said at least one second mRNA molecule, or in said single mRNA molecule, is replaced by chemically modified nucleosides, such as a N1-methyl-pseudouridine, pseudouridine (ψ), or 5-methylcytidine (m5C), and/or wherein the at least one first mRNA molecule and the at least one second mRNA molecule, or the single mRNA molecule, preferably include an optimized Kozak sequence, an optimized codon usage, optimized 5' and/or 3' untranslated sequences, a poly A tail, a 5' CAP preferentially selected from but not restricted to Anti-Reverse Cap Analog (ARCA), CAPO, CAP1: m7GpppNmpN, CAP2: m7GpppNmpNm, and/or an internal ribosomal entry site (IRES) in the context of linear or circular RNA.

11. A host cell comprising the combination or the single mRNA molecule according to any one of claims 1 to 10, optionally wherein the host cell is an antigen presenting cell, preferably a professional antigen presenting cell, or a lymphocyte, preferably a T lymphocyte or T lymphocyte progenitor, more preferably a CD4 or CD8 positive T-cell.

12. A pharmaceutical composition comprising the combination or the single mRNA molecule according to any one of claims 1 to 10, or the host cell according to claim 11, and a pharmaceutical acceptable carrier, stabilizer and/or excipient.

13. A vaccine comprising the combination or the single mRNA molecule according to any one of claims 1 to 10, the host cell according to claim 11, or the pharmaceutical composition according to claim 12, optionally wherein the vaccine elicits an adaptive immune response, preferably a protective adaptive immune response against:
(i) a virus, such as a virus selected from, but not restricted to, the following list: coronavirus, SARS coronavirus, SARS-CoV-2 coronavirus, respiratory syncytial virus (RSV), measles virus, influenza virus, rabies virus, Dengue fever virus, HIV (human immunodeficiency virus), hepatitis A virus (HAV), hepatitis B virus (HBV), hepatitis C virus (HCV), hepatitis D virus, hepatitis E virus, foot-and-mouth disease virus (FMDV), bovine leukosis virus (BLV), bovine parainfluenza virus, human parainfluenza virus (HPIV), bovine respiratory syncytial virus, porcine reproductive and respiratory syndrome virus, respiratory syncytial virus, vesicular stomatitis virus, bovine viral diarrhea virus, bovine coronavirus, BHV1 virus, equine arteritis virus, Nipah virus, BK virus, Porcine Respiratory Corona Virus Infection, Jaagsiekte Sheep Retrovirus, infectious bronchitis virus, avian pneumovirus, Calicivirus, enterovirus, Epstein-bar virus (EBV), newcastle disease virus, astrovirus, Influenzavirus A (Avian influenza) subtype H5N1, simple herpesvirus 1 (HSV-1), simple herpesvirus 2 (HSV-2), heartland virus, bocavirus (HBoV), human herpesvirus 6 (HHV-6), human herpesvirus 7 (HHV-7), human metapneumovirus (hMPV), human papilloma virus (HPV), Japanese encephalitis virus, JC virus, funin virus, Cytomegalovirus (CMV), Machupovirus, Marburg virus, infectious soft tumor virus (MCV), Lassavirus, epidemic parotitis virus, rhinovirus, rotavirus, varicella-zoster virus, Venezuelan encephalitis virus, West Nile virus, Western horse encephalitis virus, and Yellow fever virus, Filoviruses, and/or a bacterium, such as a bacterium selected from, but not restricted to, the following list: Acinetobacter, Acinetobacter baumannii, Actinobacillus, Anaplasma, Ancylostoma duodenale, hemolytic alkholderia Viralidae, Babesia, Bacillus, Bacillus anthracis, Bacillus cereus, Bacteroides, Burkholderi, Burkholderia henselae, Burkholderia mallei, Bordella pertussis, Bordetella, Brazilian burkholderia, Brucella, Campylobacter, Candida albicans, Chlamydia, Chlamydia trachomatis, Chlamydophila, Chlamydophila pneumoniae, Clostridium botulinum, Clostridium deficile, Clostridium candididium, Clostridium tetani, Corynebacterium diphtheriae, Clostridium, Clostridium burnetii, Cyanobacteria, Dientamoeba fragilis, Escherichia, Escherichia coli, Ehrlicia, Ehrlicia chafensis, Ehrlicia ewingii, Enterobacter, Enterococcus, Erwinia, Franciscella, Fusobacteria, Haemophilus, Haemophilus influenzae, Helicobacter, Hemophilus, Histoplasma capsultum, Kingera, Kingella, Kingella kingae, Klebsiella, Klebsiella granulomatis, Legionella, Legionella pneumophila, Listeria, Meningococcus, Moraxella, Mycobacterium leprae, Mycobacterium lepromatosis, Mycobacterium tuberculosis, Mycobacterium ulcerans mulcerans, Moraxella, pneumonia mycoplasma pneumoniae, Neisseria, Pasteurella, Prevotella, Proteus, Pseudomonas, Rickettsia, Salmonella, Serratia, Shigella, Staphylococcus, Streptococcus, Treponema, Vibrio, Yersinia, and ESKAPE pathogens, and/or a parasite, such as a parasite selected from, but not restricted to, the following list: Malaria, Leshmania, Cryptosoridium, Entamoeba, Taenia solium, Acaris lumbricoides, Echinococcus, Trypanosoma cruzi, Trypanosoma brucei, Schistosoma, and/or
(ii) a cancer antigen or a tumour antigen, such as a cancer antigen or a tumour antigen selected from 1A01 HLA-A/m; 1A02; 5T4; ACRBP; AFP; AKAP4; alpha-actinin-4/m; alpha-methylacyl-coenzyme_A_racemase; ANDR; ART-4; ARTCl/m; AURKB; B2MG; B3GN5; B4GN1; B7H4; BAGE-1; BASI; BCL-2; bcr/abl; beta-catenin/m; BING-4; BIRC7; BRCAl/m; BY55; calreticulin; CAMEL; CASPA; Caspase_8; cathepsin_B; cathepsin L; CD1A; CD1B; CD1C; CD1D; CD1E; CD20; CD22; CD276; CD33; CD3E; CD3Z; CD4; CD44_Isoform_1; CD44_lsoform_6; CD52; CD55; CD56; CD80; CD86; CD8A; CDC27/m; CDE30; CDK4/m; CDKN2A/m; CEA; CEAM6; CH3L2; CLCA2; CML28; CML66; COA-1/m; coactosin-like_protein; collagen_XXIII; COX-2; CP1B1; CSAG2; CT-9/BRD6; CT45A1; CT55; CTAG2_Isoform_LAGE-1A; CTAG2_lsoform_LAGE-1B; CTCFL; Cten; cyclin_B1; cyclin _D1; cyp-B; DAM-10; DEP1A; E7; EF1A2; EFTUD2/m; EGFR; EGLN3; ELF2/m; EMMPRIN; EpCam; EphA2; EphA3; ErbB3; ERBB4; ERG; ETV6; EWS; EZH2; FABP7; FCGR3A_Version_1; FCGR3A_Version_2; FGF5; FGFR2; fibronectin; FOS; FOXP3; FUT1; G250; GAGE-1; GAGE-2; GAGE-3; GAGE-4; GAGE-5; GAGE-6; GAGE7b; GAGE-8JGAGE-2D); GASR; GnT-V; GPC3; GPNMB/m; GRM3; HAGE; hepsin; Her2/neu; HLA-A2/m; homeobox _NKX3.1; HOM-TES-85; HPG1; HS71A; HS71B; HST-2; hTERT; iCE; IF2B3; IL-10; IL-13Ra2; IL2-RA; IL2-RB; IL2-RG; IL-5; IMP3; ITA5; ITB1; ITB6; kallikrein-2; kailikrein-4; KI20A; KIAA0205; KIF2C; KK-LC-1; LDLR; LGMN; LIRB2; LY6K; MAGA5; MAGA8; MAGAB; MAGE- B1; MAGE- E1; MAGE-A1; MAGE-A10; MAGE-A12; MAGE-A2; MAGE-A3; MAGE-A4; MAGE-A6; MAGE-A9; MAGE-BIO; MAGE-B16; MAGE-B17; MAGE-B2; MAGE-B3; MAGE-B4; MAGE-B5; MAGE-B6; MAGE-C1; MAGE-C2; MAGE-C3; MAGE-D1; MAGE-D2; MAGE-D4; MAGE-E1JMAGE1); MAGE-E2; MAGE-F1; MAGE-HI; MAGEL2; mammaglobin A; MART-l/melan-A; MART-2; MC1R; M-CSF; mesothelin; MITF; MMP1; MMP7; UC-1; MUM-l/m; MUM-2/m; MY01A; MY01B; MY01C; MY01D; MY01E; MY01F; MY01G; MY01H; NA17; NA88-A; Neo-PAP; NFYC/m; NGEP; N-myc; NPM; NRCAM; NSE; NUF2; NY-ESO-1; OA1; OS-9; osteocalcin; osteopontin; p53; PAGE-4; PAI-1; PAI-2; PAP; PATE; PAX3; PAX5; PD1L1; PDCD1; PDEF; PECA1; PGCB; PGFRB; Pim-1-Kinase; Pin-1; PLAC1; PMEL; PML; POTE; POTEF; PRAME; PRDX5/m; PRM2; prostein; proteinase-3; PSA; PSB9; PSCA; PSGR; PSM; PTPRC; RAB8A; RAGE-1; RARA; RASH; RASK; RASN; RGS5; RHAMM/CD168; RHOC; RSSA; RU1; RU2; RUNX1; S-100; SAGE; SART-1; SART-2; SART-3; SEPR; SERPINB5; SIA7F; SIA8A; SIAT9; SIRT2/m; SOX10; SP17; SPNXA; SPXIM3; SSX-1; SSX-2; SSX3; SSX-4; ST1A1; STAG 2; STAMP-1; STEAP-1; Survivin; Survivin-2B; SYCP1; SYT-SSX-1; SYT-SSX-2; TARP; TCRg; TF2AA; TGFbeta1; TGFR2; TGM-4; TIE2; TKTL1; TPI/m; TRGV11; TRGV9; TRPC1; TRP-p8; TSG10; TSPY1; TVCJTRGV3; TX101; tyrosinase; TYRP1; TYRP2; UPA; VEGFR1, and XAGE1; and/or a tumor neo-antigen, preferably selected from the group consisting of a single amino acid mutant peptide, a frame-shift peptide, a read-through mutation peptide, a splice site mutant peptide, and/or a senescence associated antigen.

14. A pharmaceutical combination comprising:
(i) the combination or the single mRNA molecule according to any one of claims 1 to 10, the host cell according to claim 11, the pharmaceutical composition according to claim 12, or the vaccine according to claim 13, and
(ii) a checkpoint inhibitor, optionally wherein the checkpoint inhibitor is selected from Atezolizumab, Avelumab, Cemiplimab, Dostarlimab, Durvalumab, Nivolumab, Pembrolizumab, Ipilimumab, and Relatlimab, and/or
(iii) an antagonistic antibody or an antagonistic antibody fragment or an antagonistic peptide against an immune checkpoint protein, optionally wherein the immune checkpoint protein is preferentially selected from but not restricted to any one of PD-1, cd47, cd31, PD-L1, PD-L2, BTLA, HVEM, lag3, tigit, prv, cd155, cd112, galectin-9, tim3, tim4, gitr, gitrl, tigit, Vista, cd276, cd39, cd73, CTLA4, IL10, IL35, ido, vip, IL27, and IL37.

15. A kit comprising the combination or the single mRNA molecule according to any one of claims 1 to 10, the host cell according to claim 11, the pharmaceutical composition according to claim 12, the vaccine according to claim 13, or the pharmaceutical combination to claim 14, and optionally comprising a liquid vehicle for solubilising, and further optionally technical instructions providing information on administration and/or dosage of the components.

16. The combination or the single mRNA molecule according to any one of claims 1 to 10, the host cell according to claim 11, the pharmaceutical composition according to claim 12, the vaccine according to claim 13, the pharmaceutical combination to claim 14, or the kit according to claim 15, for use in medicine.

17. The combination or the single mRNA molecule according to any one of claims 1 to 10, the host cell according to claim 11, the pharmaceutical composition according to claim 12, the vaccine according to claim 13, the pharmaceutical combination to claim 14, or the kit according to claim 15, for use in a method of diagnosis, prevention, and/or treatment of an infectious, a genetic, or a proliferative disease, wherein the infectious disease is preferably a viral infection, a bacterial infection, a fungal infection, a protozoan infection, and/or a parasite infection, such as an infection caused by a corona virus, for example a COVID-19 disease, and wherein the proliferative disease is preferably cancer, more preferably a cancer selected from pancreatic cancer, bladder cancer, breast cancer, liver cancer, lung cancer, cutaneous squamous cell carcinoma, basal cell carcinoma, melanoma, uterine (endometrial) cancer, kidney cancer, skin cancer, such as Merkel cell carcinoma, colorectal cancer, esophageal cancer, gastric cancer, head and neck cancer, neuroblastoma, leukemia, sarcoma, kidney cancer, lymphoma, mesothelioma, cervical cancer, stomach cancer, and a cancer that presents with certain genetic mutations (such as MSI-H, dMMR, or TMB-H).

18. The combination or the single mRNA molecule according to any one of claims 1 to 10, the host cell according to claim 11, the pharmaceutical composition according to claim 12, the vaccine according to claim 13, the pharmaceutical combination to claim 14, or the kit according to claim 15, for use according to claim 17, wherein said method comprises administration of the combination or the single mRNA molecule according to any one of claims 1 to 10, the host cell according to claim 11, the pharmaceutical composition according to claim 12, the vaccine according to claim 13, the pharmaceutical combination to claim 14, the kit according to claim 15, the single vector comprising the at least one first mRNA molecule and the at least one second mRNA molecule, the two separate compositions comprising the at least one first mRNA molecule and the at least one second mRNA molecule, respectively, or the combined composition, or the single mRNA molecule, to a subject, optionally wherein the single vector comprising the at least one first mRNA molecule and the at least one second mRNA molecule, or the two separate compositions comprising the at least one first mRNA molecule and the at least one second mRNA molecule, respectively, or the combined composition, or the single mRNA molecule, are administered to the subject, further optionally wherein the two separate compositions comprising the at least one first mRNA molecule and the at least one second mRNA molecule, respectively, are administered separately, and wherein
i) the two separate compositions are administered separately at a first location site and at a second location site, wherein the first location site is within 20 cm, 17.5 cm, 15 cm, 12.5 cm, 10 cm, 7.5 cm, 5 cm, 2.5 cm, 1 cm, 0.5 cm, 0.25 cm, or 0.1 cm, of the second location site, preferably wherein the draining lymphatic system of the first location site drains to the same lymph nodes as the lymphatic system of the second location site, or wherein the first location site and the second location site are the same, and/or
ii) the two separate compositions are administered separately within a time interval of 1 hour or less, 30 minutes or less, 20 minutes or less, 15 minutes or less, 10 minutes or less, 5 minutes or less, 3 minutes or less, or 1 minute or less,
to allow a spatially and/or temporally coordinated *in vivo* expression of the at least one first molecule, which is a therapeutic or immunogenic protein or peptide, and the at least one second molecule, which is a protein or a peptide having the ability to modulate the immune response against the first molecule and/or the translation product of the first molecule.

19. The combination or the single mRNA molecule according to any one of claims 1 to 10, the host cell according to claim 11, the pharmaceutical composition according to claim 12, the vaccine according to claim 13, the pharmaceutical combination to claim 14, or the kit according to claim 15, for use according to claim 17 or 18, wherein the combination or the single mRNA molecule according to any one of claims 1 to 10, the host cell according to claim 11, the pharmaceutical composition according to claim 12, the vaccine according to claim 13, the pharmaceutical combination to claim 14, the kit according to claim 15, the single vector comprising the at least one first mRNA molecule and the at least one second mRNA molecule, the two separate compositions comprising the at least one first mRNA molecule and the at least one second mRNA molecule, respectively, or the combined composition, or the single mRNA molecule, are administered to the subject by intramuscular, subcutaneous, intradermal, intra-peritoneal, intravenous, and/or intra-pleural injection, wherein, preferably, the two separate compositions comprising the at least one first mRNA molecule and the at least one second mRNA molecule, respectively, are administered by the same route.
